# EUROPEAN PATENT APPLICATION

(11) **EP 4 155 407 A1**
(43) Date of publication of application: **29.03.2023**
(21) Application number: 21807984.6
(22) Date of filing: 10.02.2021
(51) Int. Cl.: C12N 15/56, A23L 33/115, A61K 36/28, A61K 36/31, A61K 36/42, A61K 36/73, A61K 36/81, A61K 36/88, A61K 36/896, A61K 36/899, A61K 38/47, A61P 1/16, A61P 3/00, A61P 7/04, A61P 7/06, A61P 19/08, A61P 43/00, C07K 14/415, C12N 1/15, C12N 1/19, C12N 1/21

(54) **THERMOSTABLE GLUCOCEREBROSIDASE**

(30) Priority: 21.05.2020 JP 2020088950
(71) Applicant: Teikyo University, Tokyo 173-8605 (JP)
(72) Inventor: KOGA, Jinichiro, Utsunomiya-shi, Tochigi 320-8551 (JP); YAMANE, Hisakazu, Utsunomiya-shi, Tochigi 320-8551 (JP); MIYAMOTO, Koji, Utsunomiya-shi, Tochigi 320-8551 (JP); YAZAWA, Makoto, Utsunomiya-shi, Tochigi 320-8551 (JP); KUBOTA, Tomoyoshi, Utsunomiya-shi, Tochigi 320-8551 (JP)
(74) Representative: TBK
(86) International application number: PCT/JP2021/004999
(87) International publication number: WO 2021/235023

(57) **Abstract**

Provided is a protein having glucocerebrosidase activity and further having thermostability. The object is attained by a protein which is derived from a plant, belongs to glycoside hydrolase family 1 (GH1), and has glucocerebrosidase activity.

## Description

### TECHNICAL FIELD

The present invention relates to a protein having glucocerebrosidase activity and thermostability (hereinafter, this is also referred to as "thermostable glucocerebrosidase"), an enzyme composition, a pharmaceutical composition or a food composition including this protein, a method for producing ceramide using this protein, and the like.

### BACKGROUND ART

Glucocerebrosidase is known as an enzyme that converts glucosylceramide, one of the glycolipids, to ceramide through hydrolysis. This glucocerebrosidase is found to exist mainly in animals and plays an important role in generating ceramide from glucosylceramide in the animal body, though its existence is hardly known for plants.

An inborn error of metabolism that cannot convert glucosylceramide in the body to ceramide due to congenital deficiency of glucocerebrosidase gene

(Gaucher's disease) is known for humans. In this Gaucher's disease, glucosylceramide abnormally accumulates in the body so that various symptoms such as enlargement of the liver or the spleen, anemia, thrombocytopenia, and bone abnormality appear (Non-Patent Documents 1 and 2).

There reportedly exist Gaucher's disease patients equal to or more than approximately 5,000 people worldwide. Treatment that converts accumulated glucosylceramide to ceramide by replenishing scarce glucocerebrosidase with intravenous infusion drips in the body is typically performed as a treatment method therefor.

### CITATION LIST

### NON-PATENT DOCUMENTS

[Non-Patent Document 1] Annual Review of Genomics and Human Genetics 4, 403-436 (2003)
[Non-Patent Document 2] British Journal of Haematology 129, 178-188 (2005)

### SUMMARY OF THE INVENTION

### PROBLEM TO BE SOLVED BY THE INVENTION

At present, imiglucerase which is human-derived glucocerebrosidase produced in Chinese hamster ovary cells by use of a gene recombination technique is typically used as an enzyme drug for use as intravenous infusion drips for Gaucher's disease. However, a problem of this imiglucerase is large economic burdens on patients because the drug price is high and furthermore, once-every-two weeks replenishment is required due to low thermostability.

Since animal-derived glucocerebrosidase is an enzyme that acts around the body temperature of the animal, any type thereof has low thermostability, as in the imiglucerase described above. Accordingly, if glucocerebrosidase that has thermostability and can minimize the number of times of replenishment is present, its medical value is unmeasurable. However, such glucocerebrosidase has not yet been reported.

Meanwhile, ceramide present in the stratum corneum of human skin, or the like is a component necessary for keeping moisture in the skin, and is blended into cosmetics or the like. This ceramide serving as an active ingredient in cosmetics or the like is usually produced by chemical synthesis. In this context, it could be possible to produce ceramide by decomposing relatively inexpensive glucosylceramide having a high content in animals or plants with glucocerebrosidase and to use this ceramide in cosmetics, reagents, or the like. However, since the imiglucerase described above is very expensive and also has low thermostability, practical use thereof has not yet been achieved. Unlike glucosylceramide, ceramide has a very low content in animals or plants. Therefore, direct extraction and purification of this ceramide involve enormous cost.

Accordingly, an object of the present invention is to provide a protein having glucocerebrosidase activity and further having thermostability.

### MEANS FOR SOLVING THE PROBLEM

The present inventor has conducted diligent studies to attain the object and found a protein which is derived from a plant, belongs to glycoside hydrolase family 1, and has glucocerebrosidase activity. It has been further found that this protein has thermostability, completing the present invention. It should be noted that there has been no report so far stating that glucocerebrosidase belonging to GH1 has been found from plants.

Specifically, the present invention relates to the following <1> to <17>.
<1> A protein which is derived from a plant, belongs to glycoside hydrolase family 1, and has glucocerebrosidase activity.
<2> The protein according to <1>, wherein the plant is a seed plant.
<3> The protein according to <2>, wherein the seed plant is one of a plant of the family *Brassicaceae,* a plant of the family *Poaceae,* a plant of the family *Cucurbitaceae,* a plant of the family *Compositae,* a plant of the family *Solanaceae,* a plant of the family *Rosaceae,* a plant of the family *Amaryllidaceae,* a plant of the family *Leguminosae,* and a plant of the family *Liliaceae.*
<4> A protein shown in the following (A), (B), or (C):
   (A) a protein consisting of an amino acid sequence represented by SEQ ID NO: 1, amino acid positions 38 to 521 of SEQ ID NO: 1, SEQ ID NO: 2, or amino acid positions 19 to 503 of SEQ ID NO: 2 in the sequence listing.
   (B) a protein which consists of the amino acid sequence represented by SEQ ID NO: 1, amino acid positions 38 to 521 of SEQ ID NO: 1, SEQ ID NO: 2, or amino acid positions 19 to 503 of SEQ ID NO: 2 in the sequence listing, having one or several nucleotides substituted, deleted, inserted, or added, belongs to glycoside hydrolase family 1, and has glucocerebrosidase activity and thermostability.
   (C) a protein which has homology equal to or more than 60% to the amino acid sequence represented by SEQ ID NO: 1, amino acid positions 38 to 521 of SEQ ID NO: 1, SEQ ID NO: 2, or amino acid positions 19 to 503 of SEQ ID NO: 2 in the sequence listing, belongs to glycoside hydrolase family 1, and has glucocerebrosidase activity and thermostability.
<5> DNA encoding a protein according to any one of <1> to <4>.
<6> DNA shown in the following (a), (b), or (c):
   (a) DNA which consists of a nucleotide sequence represented by SEQ ID NO: 3, nucleotide positions 112 to 1566 of SEQ ID NO: 3, SEQ ID NO: 4, or nucleotide positions 55 to 1512 of SEQ ID NO: 4 in the sequence listing, and encodes a protein belonging to glycoside hydrolase family 1 and having glucocerebrosidase activity.
   (b) DNA which consists of the nucleotide sequence represented by SEQ ID NO: 3, nucleotide positions 112 to 1566 of SEQ ID NO: 3, SEQ ID NO: 4, or nucleotide positions 55 to 1512 of SEQ ID NO: 4 in the sequence listing, having one or several nucleotides substituted, deleted, inserted, or added, and encodes a protein belonging to glycoside hydrolase family 1 and having glucocerebrosidase activity and thermostability.
   (c) DNA which is capable of hybridizing under stringent conditions to DNA consisting of a nucleotide sequence complementary to the nucleotide sequence represented by SEQ ID NO: 3, nucleotide positions 112 to 1566 of SEQ ID NO: 3, SEQ ID NO: 4, or nucleotide positions 55 to 1512 of SEQ ID NO: 4 in the sequence listing, and encodes a protein belonging to glycoside hydrolase family 1 and having glucocerebrosidase activity and thermostability.
<7> An expression vector for the expression of a protein belonging to glycoside hydrolase family 1 and having glucocerebrosidase activity and thermostability, the expression vector including DNA according to <5> or <6>.
<8> A transformant harboring an expression vector according to <7>.
<9> The transformant according to <8>, wherein the transformant is one of a plant, a plant cell, an animal cell, *Escherichia coli,* a yeast, and a filamentous fungus.
<10> A method for producing a protein, including the steps of: breeding or culturing a transformant according to <8> or <9>; and recovering a protein according to any one of <1> to <4> from the transformant or a material containing the transformant obtained by the step.
<11> An enzyme composition for glucosylceramide hydrolysis, containing a protein according to any one of <1> to <4>.
<12> A pharmaceutical composition including a protein according to any one of <1> to <4> as an active ingredient.
<13> The pharmaceutical composition according to <12> for Gaucher's disease prevention and treatment.
<14> A food composition containing a protein according to any one of <1> to <4>, and glucosylceramide isolated from a plant, an animal, or a microbe, or chemically synthesized glucosylceramide.
<15> A method for producing ceramide, including the step of using a protein according to any one of <1> to <4> to generate ceramide from glucosylceramide isolated from a plant, an animal, or a microbe, or chemically synthesized glucosylceramide.
<16> A method for preventing or treating Gaucher's disease, including administering (by intravenous infusion or the like) a protein according to any one of <1> to <4> or a pharmaceutical composition according to <12> or <13> to a Gaucher's disease patient.
<17> The method for preventing or treating Gaucher's disease according to <16>, wherein the protein or the pharmaceutical composition is administered to the Gaucher's disease patient once per 3 to 10 weeks such that glucocerebrosidase activity is 10 to 200 U/kg body weight.

### EFFECT OF THE INVENTION

The present invention can provide a protein belonging to glycoside hydrolase family 1 and having glucocerebrosidase activity and thermostability. Furthermore, an enzyme composition, a pharmaceutical composition, and a food composition comprising this protein can also be provided. Moreover, a method for producing ceramide using this protein can also be provided.

### BRIEF DESCRIPTION OF THE DRAWINGS

[Fig. 1] Fig. 1 is a graph showing the relationship between an incubation time at 37°C (pH 5) and relative residual activity for rice-derived glucocerebrosidase (RGC1) and human-derived glucocerebrosidase (imiglucerase).
[Fig. 2] Fig. 2 is a graph showing the relationship between an incubation time at 37°C (pH 7) and relative residual activity for rice-derived glucocerebrosidase (RGC1) and human-derived glucocerebrosidase (imiglucerase).
[Fig. 3] Fig. 3 is a graph showing the relationship between an incubation time at 45°C (pH 5) and relative residual activity for soybean-derived glucocerebrosidase (SGC1) and human-derived glucocerebrosidase (imiglucerase).
[Fig. 4] Fig. 4 is a graph showing the relationship between an incubation time at 45°C (pH 7) and relative residual activity for soybean-derived glucocerebrosidase (SGC1) and human-derived glucocerebrosidase (imiglucerase).
[Fig. 5] Fig. 5 shows the amino acid sequence of rice-derived glucocerebrosidase (RGC1).
[Fig. 6] Fig. 6 shows the nucleotide sequence of DNA encoding the rice-derived glucocerebrosidase (RGC1).
[Fig. 7] Fig. 7 shows the amino acid sequences of 9 peptide fragments obtained by the trypsin treatment of the rice-derived glucocerebrosidase (RGC1).
[Fig. 8] Fig. 8 shows the nucleotide sequences of a F-primer and a R-primer used in the cloning of the DNA encoding the rice-derived glucocerebrosidase (RGC1).
[Fig. 9] Fig. 9 shows the amino acid sequence of soybean-derived glucocerebrosidase (SGC1).
[Fig. 10] Fig. 10 shows the nucleotide sequence of DNA encoding the soybean-derived glucocerebrosidase (SGC1).
[Fig. 11] Fig. 11 shows the amino acid sequences of 7 peptide fragments obtained by the trypsin treatment of the soybean-derived glucocerebrosidase (SGC1).
[Fig. 12] Fig. 12 shows the nucleotide sequences of a F-primer and a R-primer used in the cloning of the DNA encoding the soybean-derived glucocerebrosidase (SGC1).

### DESCRIPTION OF EMBODIMENTS

The present invention will be described.

The present invention relates to a protein which is derived from a plant, belongs to glycoside hydrolase family 1, and has glucocerebrosidase activity (hereinafter, this is also referred to as the "protein of the present invention" or the "thermostable glucocerebrosidase of the present invention"), DNA encoding the protein of the present invention and an expression vector including this DNA, a transformant harboring this expression vector and a method for producing the protein of the present invention using this transformant, an enzyme composition, a pharmaceutical composition or a food composition including the protein of the present invention, a method for producing ceramide using the protein of the present invention, and the like.

First, the protein of the present invention will be described in detail.

The protein of the present invention is a protein which is derived from a plant, belongs to glycoside hydrolase family 1 (GH1), and has glucocerebrosidase activity. This protein is derived from a plant, belongs to GH1, and has glucocerebrosidase activity has thermostability.

In this context, the "glucocerebrosidase activity" is the enzymatic activity of an enzyme with EC No. of (EC3.2.1.45) (glucocerebrosidase), that is, the activity of catalyzing reaction of hydrolyzing the β-1,4-glycosyl bond between glucose and ceramide of the glycolipid glucosylceramide to generate ceramide. The "glycoside hydrolase family 1" is one of the carbohydrate hydrolase families classified into approximately 130 families according to Carbohydrate Active enzyme database (CAZy databe, http://www.cazy.org/). Glucocerebrosidase belonging to glycoside hydrolase family 1 (GH1), glucocerebrosidase belonging to glycoside hydrolase family 30 (GH30), and glucocerebrosidase belonging to glycoside hydrolase family 116 (GH116) are known.

The phrase "derived from a plant" means that the protein is expressed from a gene of the plant. This gene of the plant can be obtained from at least one selected from the group consisting of, for example, a leaf, a stem, a root, a seed, a fruit, a petal, a pistil, a pollen (stamen), a rhizoid, and a sporangium of the plant.

The "thermostability" means performance by which, when a solution containing a protein having glucocerebrosidase activity with glucocerebrosidase activity of 0.0002 to 0.0008 U/mL and a 50 mM acetate buffer solution (pH 5.0) that contain 0.1% Triton X-100 (registered trademark; the same holds true for the description below) and 0.05% sodium cholate is incubated at 37°C for 30 hours, this glucocerebrosidase activity equal to or more than 80% remains (the relative residual activity equal to or more than 80% based on the glucocerebrosidase activity before incubation defined as 100%). For this thermostability, it is preferred that the relative residual activity described above be equal to or more than 90%. In this context, this glucocerebrosidase activity is measured as follows: a predetermined amount of sample is added to a 50 mM acetate buffer solution (pH 5.5) containing 100 µM glucosylceramide (cerebroside B, (4E,8E)-N-D-2'-hydroxypalmitoyl-1-*O*-β-D-glucopyranosyl-9-methyl-4,8-sphingadienine (The Journal of Antibiotics 41, (1988) 469-480; the same holds true for the description below), 0.1% Tween 20, and 0.05% sodium cholate, and incubated at 37°C for 15 to 60 minutes. Subsequently, a 4-fold amount of ethanol is added to the obtained enzymatic reaction solution, mixed therewith, and then centrifuged at 15000 rpm for 20 minutes. The supernatant is subjected to high-performance liquid chromatography analysis to measure the amount of ceramide generated through enzymatic reaction. Conditions, other than those described above, necessary for the glucocerebrosidase activity measurement are carried out in accordance with a method described in Example 2 mentioned later.

The protein of the present invention is preferably derived from a seed plant. Specific examples of the seed plant include, but are not limited to, plants of the family *Malvaceae,* plants of the family *Chenopodiaceae,* plants of the family *Rubiaceae,* plants of the family *Cannabaceae,* plants of the family *Hydrangeaceae,* plants of the family *Brassicaceae,* plants of the family *Iridaceae,* plants of the family *Poaceae,* plants of the family *Araliaceae,* plants of the family *Cucurbitaceae,* plants of the family *Anacardiaceae,* plants of the family *Cyperaceae,* plants of the family *Campanulaceae,* plants of the family *Compositae,* plants of the family *Lauraceae,* plants of the family *Moraceae,* plants of the family *Papaveraceae,* plants of the family *Araceae,* plants of the family *Cactaceae,* plants of the family *Lamiaceae,* plants of the family *Nymphaeaceae,* plants of the family *Apiaceae,* plants of the family *Polygonaceae,* plants of the family *Ericaceae,* plants of the family *Theaceae,* plants of the family *Solanaceae,* plants of the family *Caryophyllaceae,* plants of the family *Ulmaceae,* plants of the family *Nelumbonaceae,* plants of the family *Rosaceae,* plants of the family *Amaryllidaceae,* plants of the family *Convolvulaceae,* plants of the family *Vitaceae,* plants of the family *Fagaceae,* plants of the family *Paeoniaceae,* plants of the family *Leguminosae,* plants of the family *Rutaceae,* plants of the family *Pontederiaceae,* plants of the family *Oleaceae,* plants of the family *Arecaceae,* plants of the family *Salicaceae,* plants of the family *Liliaceae,* plants of the family *Orchidaceae,* plants of the family *Taxaceae,* plants of the family *Ginkgoaceae,* plants of the family *Taxodiaceae,* plants of the family *Cycadaceae,* plants of the family *Cupressaceae,* and plants of the family *Pinaceae.*

Examples of the seed plant more specifically include cotton plant, hibiscus, spinach, fat hen, beet, madder, gardenia, coffee tree, marijuana, hop, hydrangea, Arabidopsis thaliana, rapeseed, Japanese radish, napa, cabbage, cauliflower, broccoli, Japanese mustard spinach, qing-geng-cai, wasabi, water iris, Japanese iris, iris, rice, timothy, wheat, maize, sorghum, barley, rye, sugar cane, oat, Japanese millet, foxtail millet, Korean lawn grass, common reed grass, bamboo, arrow bamboo, Japanese angelica tree, udo, fatsia, cucumber, bitter cucumber, melon, watermelon, bitter gourd, pumpkin, loofa, winter melon, gourd, calabash, lacquer tree, Japanese wax tree, star grass, balloon flower, goldenmane tickseed, lettuce, gerbera, gazania, thistle, burdock, sunflower, cosmos, dandelion, calendula, sweet coltsfoot, annual ragweed, camphor tree, bay tree, mulberry, fig, common poppy, water lettuce, eddo, cactus, Japanese basil, salvia, lavender, water lily, carrot, water celery, celery, buckwheat, polygonum, rhododendron, blueberry, alpine rose, camellia, tomato, eggplant, tobacco, petunia, capsicum, potato, dianthus, carnation, annual baby's breath, chickweed, Japanese zelkova, Muku tree, lotus, rose, cherry, almond, apricot, strawberry, Japanese apricot, apple, nashi pear, loquat, peach, garlic, green onion, onion, false bindweed, morning glory, sweet potato, grape, Japanese beech, jolcham oak, sawthorn oak, Japanese chestnut, tree peony, soybean, broad bean, black bean, Japanese wisteria, Japanese wisteria, lupine, kidney bean, pea, alfalfa, peanut, sweet pea, bird's-foot trefoil, miyagawa mandarin, Japanese pepper, Japanese summer orange, orange, lime, lemon, grapefruit, hardy orange, water hyacinth, olive, common jasmine, coconut palm, oil palm, Chinese date, chusan palm, poplar, basket willow, lily, morning star lily, tulip, narcissus, moth orchid, cattleya, vanilla, yew, gingko, cedar, cycad, Japanese cypress, and pine.

Among them, a protein that is derived from one of a plant of the family *Brassicaceae,* a plant of the family *Poaceae,* a plant of the family *Cucurbitaceae,* a plant of the family *Compositae,* a plant of the family *Solanaceae,* a plant of the family *Rosaceae,* a plant of the family *Amaryllidaceae,* a plant of the family *Leguminosae,* and a plant of the family *Liliaceae,* belongs to GH1, and has glucocerebrosidase activity is particularly suitable because of its excellent thermostability. For example, a rice-derived or soybean-derived protein that belongs to GH1 and has glucocerebrosidase activity has very suitable glucocerebrosidase activity and thermostability.

The rice-derived thermostable glucocerebrosidase of the present invention consists of the amino acid sequence represented by SEQ ID NO: 1 in the sequence listing, or the amino acid sequence represented by amino acid positions 38 to 521 of this SEQ ID NO: 1 which is an amino acid sequence from which a signal peptide has been cleaved off. The soybean-derived thermostable glucocerebrosidase of the present invention consists of the amino acid sequence represented by SEQ ID NO: 2 in the sequence listing, or the amino acid sequence represented by amino acid positions 19 to 503 of this SEQ ID NO: 2 which is an amino acid sequence from which a signal peptide has been cleaved off.

A protein which consists of the amino acid sequence represented by one of SEQ ID NO: 1 in the sequence listing, amino acid positions 38 to 521 of this SEQ ID NO: 1, SEQ ID NO: 2 in the sequence listing, and amino acid positions 19 to 503 of this SEQ ID NO: 2, having one or several nucleotides substituted, deleted, inserted, or added, belongs to GH1, and has glucocerebrosidase activity and thermostability is also encompassed in the present invention. In this case, this protein is preferably derived from a plant and may not be derived from a plant (the "protein of the present invention" mentioned later may include the one that is not derived from a plant). In this context, the term "several" means equal to or less than 10 and is preferably equal to or less than 6, more preferably equal to or less than 5.

This substitution, deletion, insertion, or addition of amino acids is conservative. That is, one or several amino acid residues are substituted, deleted, inserted, or added so as not to substantially alter the properties of the protein. Examples thereof include the case of substituting a hydrophobic amino acid residue with another hydrophobic amino acid residue, and the case of substituting a polar amino acid residue with another polar amino acid residue having the same charge thereas. Examples of such functionally similar amino acids specifically include hydrophobic (nonpolar) amino acids such as alanine, valine, isoleucine, leucine, proline, tryptophan, phenylalanine, and methionine. Among polar amino acids, examples of neutral amino acids include glycine, serine, threonine, tyrosine, glutamine, asparagine, and cysteine. Examples of basic amino acids include arginine, histidine, and lysine. Examples of acidic amino acids include aspartic acid and glutamic acid.

A protein which has homology equal to or more than 60%, more preferably equal to or more than 62%, further preferably equal to or more than 65%, further preferably equal to or more than 67%, further preferably equal to or more than 70%, further preferably equal to or more than 73%, further preferably equal to or more than 77%, to the amino acid sequence represented by one of SEQ ID NO: 1, amino acid positions 38 to 521 of SEQ ID NO: 1, SEQ ID NO: 2, and amino acid positions 19 to 503 of SEQ ID NO: 2 in the sequence listing, belongs to GH1, and has glucocerebrosidase activity and thermostability is also encompassed in the present invention. In this case as well, this protein is preferably derived from a plant and may not be derived from a plant. In this context, the "homology" is a numeric value calculated using default (initial setting) parameters in the homology search program EMBOSS Needle (https://www.ebi.ac.uk/Tools/psa/emboss_needle/).

Next, DNA encoding the protein of the present invention, and a method for producing the protein using this DNA will be described in detail.

The DNA encoding the protein of the present invention may be naturally derived or synthesized using a portion of the naturally derived one, for example, without limitations as long as it is constituted by a nucleotide sequence that permits expression of this protein. For example, DNA consisting of a nucleotide sequence represented by SEQ ID NO: 3 or nucleotide positions 112 to 1566 of SEQ ID NO: 3 in the sequence listing is disclosed as DNA encoding the rice-derived thermostable glucocerebrosidase of the present invention mentioned above. DNA consisting of a nucleotide sequence represented by SEQ ID NO: 4 or nucleotide positions 55 to 1512 of SEQ ID NO: 4 in the sequence listing is disclosed as DNA encoding the soybean-derived thermostable glucocerebrosidase of the present invention mentioned above.

Further examples thereof also include DNA which consists of the nucleotide sequence represented by one of SEQ ID NO: 3, nucleotide positions 112 to 1566 of SEQ ID NO: 3, SEQ ID NO: 4, and nucleotide positions 55 to 1512 of SEQ ID NO: 4 in the sequence listing, having one or several nucleotides substituted, deleted, inserted, or added, and encodes a protein belonging to GH1 and having glucocerebrosidase activity and thermostability. In this context, the term "several" means equal to or less than 20 and is preferably equal to or less than 10, more preferably equal to or less than 6.

Further examples thereof also include DNA which is capable of hybridizing under stringent conditions to DNA consisting of a nucleotide sequence complementary to the nucleotide sequence represented by one of SEQ ID NO: 3, nucleotide positions 112 to 1566 of SEQ ID NO: 3, SEQ ID NO: 4, and nucleotide positions 55 to 1512 of SEQ ID NO: 4 in the sequence listing, and encodes a protein belonging to GH1 and having glucocerebrosidase activity and thermostability.

In this context, the "stringent conditions" are conditions under which a so-called specific hybrid is formed and a nonspecific hybrid is not formed. One example thereof includes conditions involving performing hybridization at 65°C in the presence of 0.7 to 1.0 M sodium chloride, and then performing washing one to three times at 60°C, preferably 65°C, more preferably 68°C, using a solution of 0.1 to 5 × SSC and 0.1% SDS (composition of 1 × SSC: 150 mM sodium chloride and 15 mM sodium citrate).

The present invention also provides an expression vector that is capable of replicating in a plant, a plant cell, an animal cell (also including an insect cell), or a microbe serving as a host and includes the DNA mentioned above in a state that permits expression of the protein encoded thereby. That is, an expression vector for the expression of a protein that belongs to GH1 and has glucocerebrosidase activity and thermostability is also provided. This expression vector can be constructed using, for example, a self-replicating vector which resides extrachromosomally in an independent form in a cell of a host and replicates in a manner independent of the replication of chromosomal DNA, or a vector that is integrated into the chromosomal DNA of a cell of a host and replicates together with this chromosomal DNA. Preferred examples thereof include plasmid vectors and virus vectors. A method that is routinely used in the field of gene engineering can be used as procedures and a method for constructing the expression vector.

This expression vector preferably contains, in addition to the DNA encoding the protein of the present invention, a nucleotide sequence that controls expression thereof, a gene marker for the selection of a transformant, and the like in order to express the protein of the present invention in a transformant actually harboring this. Examples of the nucleotide sequence that controls expression include promoters, terminators, and nucleotide sequences encoding signal peptides other than those mentioned above. The promoter is not particularly limited as long as it exhibits transcriptional activity in a host. The signal peptide is not particularly limited as long as it contributes to the secretion of the protein to the outside of a cell in a host, for example. The gene marker can be appropriately selected according to a method for selecting a transformant. For example, a drug resistance gene or a gene that compensates for auxotrophy can be used.

The present invention also provides a transformant harboring the expression vector described above in a cell and/or chromosomal DNA of a host. This host-vector system is not particularly limited. For example, a system using a plant, a plant cell, an animal cell, or a microbe (*Escherichia coli,* yeast, filamentous fungus, or the like), or a fusion protein expression system with another protein using one of them can be used. The transfer of the expression vector described above to a host, that is, the transformation of a host using the expression vector described above, can be carried out in accordance with a method that is routinely used in the art. In this context, any of a plant, a plant cell, an animal cell, *Escherichia coli,* a yeast, and a filamentous fungus is preferably used as the host to be transformed. That is, a transformant of any of a plant, a plant cell, an animal cell, *Escherichia coli,* a yeast, and a filamentous fungus harboring the expression vector described above is preferred. For example, the transformant of a plant, a plant cell, or *Escherichia coli* harboring the expression vector described above can be allowed to express the protein of the present invention in a large amount.

This transformant is bred or cultured under conditions that permit growth and proliferation with its trait maintained, and the protein of the present invention can be recovered from the obtained transformant (plant, cultured plant cell, cultured animal cell, or the like) or material containing it (culture solution, solid medium, or the like containing the cultured cell). Thus, the present invention provides a method for producing the protein of the present invention, including the steps of: breeding or culturing the transformant; and recovering (crudely processing or purifying) the protein of the present invention from a transformant or a material containing it obtained by the step. A method for breeding or culturing the transformant and conditions therefor can be a method and conditions that permit growth and proliferation of the transformant with its trait maintained, without particular limitations, and may be substantially equivalent to a breeding or culture method and conditions as to the plant, the plant cell, the animal cell, or the microbe for use as a host. A crude processing method or a purification method that is routinely used in the art can also be used as a method for recovering the protein of interest after breeding or culture of the transformant.

One example of a preferred embodiment of the method for producing the protein of the present invention includes a method using a transformant of a plant, a plant cell, or an animal cell. Examples of the animal cell include Chinese hamster and human cells. Examples of the plant or the plant cell include plants of the family *Malvaceae,* plants of the family *Chenopodiaceae,* plants of the family *Rubiaceae,* plants of the family *Cannabaceae,* plants of the family *Hydrangeaceae,* plants of the family *Brassicaceae,* plants of the family *Iridaceae,* plants of the family *Poaceae,* plants of the family *Araliaceae,* plants of the family *Cucurbitaceae,* plants of the family *Anacardiaceae,* plants of the family *Cyperaceae,* plants of the family *Campanulaceae,* plants of the family *Compositae,* plants of the family *Lauraceae,* plants of the family *Moraceae,* plants of the family *Papaveraceae,* plants of the family *Araceae,* plants of the family *Cactaceae,* plants of the family *Lamiaceae,* plants of the family *Nymphaeaceae,* plants of the family *Apiaceae,* plants of the family *Polygonaceae,* plants of the family *Ericaceae,* plants of the family *Theaceae,* plants of the family *Solanaceae,* plants of the family *Caryophyllaceae,* plants of the family *Ulmaceae,* plants of the family *Nelumbonaceae,* plants of the family *Rosaceae,* plants of the family *Amaryllidaceae,* plants of the family *Convolvulaceae,* plants of the family *Vitaceae,* plants of the family *Fagaceae,* plants of the family *Paeoniaceae,* plants of the family *Leguminosae,* plants of the family *Rutaceae,* plants of the family *Pontederiaceae,* plants of the family *Oleaceae,* plants of the family *Arecaceae,* plants of the family *Salicaceae,* plants of the family *Liliaceae,* plants of the family *Orchidaceae,* plants of the family *Taxaceae,* plants of the family *Ginkgoaceae,* plants of the family *Taxodiaceae,* plants of the family *Cycadaceae,* plants of the family *Cupressaceae,* and plants of the family *Pinaceae,* and their cells. Examples thereof more specifically include cotton plant, hibiscus, spinach, fat hen, beet, madder, gardenia, coffee tree, marijuana, hop, hydrangea, Arabidopsis thaliana, rapeseed, Japanese radish, napa, cabbage, cauliflower, broccoli, Japanese mustard spinach, qing-geng-cai, wasabi, water iris, Japanese iris, iris, rice, timothy, wheat, maize, sorghum, barley, rye, sugar cane, oat, Japanese millet, foxtail millet, Korean lawn grass, common reed grass, bamboo, arrow bamboo, Japanese angelica tree, udo, fatsia, cucumber, bitter cucumber, melon, watermelon, bitter gourd, pumpkin, loofa, winter melon, gourd, calabash, lacquer tree, Japanese wax tree, star grass, balloon flower, goldenmane tickseed, lettuce, gerbera, gazania, thistle, burdock, sunflower, cosmos, dandelion, calendula, sweet coltsfoot, annual ragweed, camphor tree, bay tree, mulberry, fig, common poppy, water lettuce, eddo, cactus, Japanese basil, salvia, lavender, water lily, carrot, water celery, celery, buckwheat, polygonum, rhododendron, blueberry, alpine rose, camellia, tomato, eggplant, tobacco, petunia, capsicum, potato, dianthus, carnation, annual baby's breath, chickweed, Japanese zelkova, Muku tree, lotus, rose, cherry, almond, apricot, strawberry, Japanese apricot, apple, nashi pear, loquat, peach, garlic, green onion, onion, false bindweed, morning glory, sweet potato, grape, Japanese beech, jolcham oak, sawthorn oak, Japanese chestnut, tree peony, soybean, broad bean, black bean, Japanese wisteria, Japanese wisteria, lupine, kidney bean, pea, alfalfa, peanut, sweet pea, bird's-foot trefoil, miyagawa mandarin, Japanese pepper, Japanese summer orange, orange, lime, lemon, grapefruit, hardy orange, water hyacinth, olive, common jasmine, coconut palm, oil palm, Chinese date, chusan palm, poplar, basket willow, lily, morning star lily, tulip, narcissus, moth orchid, cattleya, vanilla, yew, gingko, cedar, cycad, Japanese cypress, pine, and their cells.

Another example of a preferred embodiment of the method for producing the protein of the present invention includes a method using a transformant of an *Escherichia coli* cell, a yeast cell, or a filamentous fungus cell. Examples of the yeast cell include cells of microbes belonging to the genus *Saccharomyces,* the genus *Hansenula,* or the genus *Pichia,* for example, *Saccharomyces cerevisiae.* Examples of the filamentous fungus cell include cells of ones belonging to the genus *Humicola,* the genus *Trichoderma,* the genus *Staphylotrichum,* the genus *Aspergillus,* the genus *Fusarium,* or the genus *Acremonium.*

Next, an enzyme composition, a pharmaceutical composition, and a food composition including the protein of the present invention, and the like will be described in detail.

The present invention can provide an enzyme composition containing the protein of the present invention mentioned above. This enzyme composition includes the protein of the present invention as an active ingredient (active component of the glucocerebrosidase activity of the enzyme composition) and can be suitably used for glucosylceramide hydrolysis (for the hydrolysis of glucose in a glucosylceramide molecule), that is, for the conversion of glucosylceramide to ceramide.

The protein of the present invention can be included as an active ingredient to thereby provide a pharmaceutical composition. This pharmaceutical composition can be suitably used for the prevention and treatment of Gaucher's disease. Its form is preferably intravenous infusion drips and may be an oral drug (tablet, powder, syrup, or the like).

In other words, the present invention can provide a method for preventing or treating Gaucher's disease, including administering (for example, by intravenous infusion) a pharmaceutical composition including the protein of the present invention as an active ingredient to a Gaucher's disease patient. Its dosage and administration suitably involve administering the protein of the present invention or the pharmaceutical composition (prophylactic and therapeutic agent for Gaucher's disease) including the protein of the present invention as an active ingredient to a Gaucher's disease patient once per 3 to 10 weeks, more preferably once per 4 to 8 weeks, such that glucocerebrosidase activity is 10 to 200 U/kg body weight. In this case, the term "U (unit)" is a unit at which 1 pmol of a synthetic substrate p-nitrophenyl-β-D-glucopyranoside is decomposed at 37°C for 1 minute.

The protein of the present invention and glucosylceramide isolated from a plant, an animal, or a microbe (for example, basidiomycete) or chemically synthesized glucosylceramide can be included to thereby provide a food composition that can easily liberate ceramide from the glucosylceramide in the composition. Furthermore, the protein of the present invention and the glucosylceramide can also be included as an active ingredient to thereby provide a food composition for at least one purpose selected from improvement in skin moisture, prevention of skin damage caused by ultraviolet ray, prevention of inflammatory bowel disease, and prevention of colorectal cancer as a functional food in which this ceramide liberated serves as a functional material. Moreover, a food composition for the prevention of lifestyle-related disease (e.g., diabetes mellitus, heart disease, hypertension, and hyperlipemia) can also be provided.

According to the present invention, the thermostable glucocerebrosidase of the present invention can also be included as an active ingredient to thereby provide a food composition for Gaucher's disease prevention or treatment as a functional food, or a food composition for ceramide supplementation which is ingested with a food composition in which glucosylceramide is included. The dosage and administration of the food composition for Gaucher's disease described above can be the same as those of the pharmaceutical composition for the prevention and treatment of Gaucher's disease mentioned above.

Next, a method for producing ceramide using the protein of the present invention will be described in detail.

The method for producing ceramide using the protein of the present invention includes the step of using the protein of the present invention to generate ceramide from glucosylceramide isolated from a plant, an animal, or a microbe (for example, basidiomycete) or chemically synthesized glucosylceramide. This production method may include an arbitrary step other than those described above without largely influencing the effect of the present invention.

Glucosylceramide is relatively abundant in organisms such as animals or plants, as compared with ceramide. Ceramide can be produced at low cost from such glucosylceramide by using the thermostable glucocerebrosidase of the present invention which is inexpensively obtained by expression in a large amount by the transformant mentioned above. Furthermore, the ceramide thus obtained can be used to inexpensively provide a cosmetic or a functional food aimed at an improving effect on the retention of skin moisture, a preventive effect on skin damage caused by ultraviolet ray, a preventive effect on colorectal cancer, or the like. Moreover, ceramide as a reagent for research or a drug can also be inexpensively provided.

The embodiments described above are mere examples for facilitating understanding of the present invention and do not limit the present invention.

Hereinafter, Examples of the present invention will be described. However, the present invention is not limited by Examples given below, and various changes or modifications can be made within the technical brief of the present invention.

### [Examples]

### (Example 1)

10 g of young leaves of rice (*Oryza sativa* L. cv. Nipponbare) was collected and homogenized with a homogenizer in a 50 mM acetate buffer solution (pH 5.5) containing 0.05% sodium cholate and 0.3% Triton X-100. This homogenate was centrifuged for 20 minutes under conditions of 15000 rpm and 4°C. The centrifuged supernatant was dialyzed using a 1000-fold amount of a 40 mM sodium acetate buffer solution (pH 5.5) containing 0.05% Tween 20 (registered trademark; the same holds true for the description below) and 0.025% sodium cholate to obtain an enzyme extract. To 50 mL of this enzyme extract, 10 mM glucosylceramide (cerebroside C, (4*E*,8*E*)-*N*-D-2'-hydroxy-(*E*)-3'-octadecenoyl-1-*O*-β-D-glucopyranosyl-9-methyl-4,8-sphingadienine (The Journal of Antibiotics 41,(1988)469-480; the same holds true for the description below) dissolved in 1 mL of ethanol, and 49 mL of 0.4% sodium cholate were added, followed by reaction at 45°C for 5 hours. The obtained reaction solution was adjusted to pH 11.5 with a sodium carbonate solution and a sodium hydroxide solution. Then, an ethyl acetate solution was added and mixed therewith, and centrifuged for 20 minutes under conditions of 3000 rpm and 4°C. After the centrifugation, the obtained ethyl acetate layer was dried. Then, this dried product was dissolved in an 80% ethanol solution and subjected to high-performance liquid chromatography analysis. Specifically, the sample was injected to TSKgel ODS-120T column (4.6 mm × 30 cm, manufactured by Tosoh Corp., registered trademark (the same holds true for the description below)), and a solvent having an ethanol concentration of 81% was flowed at a flow rate of 1.0 mL/min, followed by detection in a UV detector (ultraviolet absorption wavelength: 215 nm) to thereby separate a substance newly generated through enzymatic reaction.

Then, IR (FTS-135, manufactured by Bio-Rad Laboratories, Inc.), ¹H NMR, ¹³C NMR (Varian UNITY plus 500 spectrometer, manufactured by Agilent Technologies, Inc.), and ESI-MS analysis (Agilent 6460, manufactured by Agilent Technologies, Inc.) of the separated substance were conducted. The results are shown in Table 1 below.

**[Table 1]**

| |
|---|
| **IR** |
| **Vₘₐₓ 2921, 2851, 1733, 1650, 1538, 1468, 1379, 1288, 1124, 1076, 1033, 961 cm⁻¹** |
| **¹H NMR (CD₃OD)** |
| ***δ* 0.90 (6H, t, J=6.87 Hz, Me-18, 18'), 1.29 (34H, m, H-12-17, H-7'-17'), 1.37-1.42 (4H, m, H-11, 6'), 1.60 (3H, s, Me-19), 1.98 (2H, m, H-10), 2.02 (2H, m, H-5'), 2.02 (2H, m, H-6), 2.08 (2H, m, H-7), 3.67 (1H, dd, J=10.83, 3.66 Hz, H-1), 3.79 (1H, dd, J=10.83, 5.19 Hz, H-1), 3.83 (1H, m, J=7.02, 5.19 Hz, H-2), 4.07 (1H, t, J=7.17, 7.02 Hz, H-3), 4.43 (1H, d, J=6.26 Hz, H-2'), 5.14 (1H, t, J=6.71 Hz, H-8), 5.47 (1H, dd, J=15.41, 7.17 Hz, H-4), 5.50 (1H, dd, J=15.26, 6.26 Hz, H-3'), 5.71 (1H, dt, J=15.26 Hz, H-4'), 5.83 (1H, dt, J=15.41, 6.87 Hz, H-5)** |
| **¹³C NMR (125 MHz, CD₃OD)** |
| ***δ* 14.5 [C-18, -18'], 16.2 [C-19], 23.8 [C-17, -17'], 28.8 [C-7], 29.2 [C-11], 30.3-30.9 [C-12-15, C-6'~15'], 33.1 [C-16, -16'], 33.5 [C-5'], 33.8 [C-6], 40.8 [C-10], 56.1 [C-2], 61.9 [C-1], 73.4 [C-3], 74.1 [C-2'], 124.9 [C-8], 129.2 [C-3'], 131.2 [C-4], 134.4 [C-5], 134.9 [C-4'], 136.8 [C-9], 175.5 [C-1'] Parts per million from TMS using center peak of CD₃OD at 49.0ppm.** |
| **Negative ESI-MS m/z: 590.5 [M-H]⁻** |

The results revealed that the substance generated from cerebroside C by the rice-derived enzyme described above was ceramide derived from the cerebroside C. This substance was completely consistent in terms of the IR, ¹H NMR, ¹³C NMR, and ESI-MS analysis results with ceramide generated from cerebroside C by imiglucerase (manufactured by Sanofi S.A.), human-derived glucocerebrosidase. These results revealed that this rice-derived enzyme had glucocerebrosidase activity.

### (Example 2)

110 g of stems of rice (*Oryza sativa* L. cv. Nipponbare) was collected, chopped, and then homogenized with a homogenizer in a 20 mM acetate buffer solution (pH 5.5) containing 0.05% sodium cholate. This homogenate was centrifuged for 60 minutes under conditions of 10000 rpm and 4°C. The centrifuged supernatant was removed. Further, a 20 mM acetate buffer solution (pH 5.5) containing 0.05% sodium cholate was added to the centrifuged precipitate, stirred for 5 minutes, and then centrifuged for 60 minutes under conditions of 10000 rpm and 4°C. The centrifuged supernatant was removed. Further, a 20 mM acetate buffer solution (pH 5.5) containing 0.05% sodium cholate was added to the centrifuged precipitate, and Triton X-100 was added thereto in an amount sufficient for the elution of glucocerebrosidase. After stirring for 30 minutes, centrifugation was performed for 60 minutes under conditions of 18000 rpm and 4°C. The obtained centrifuged supernatant was filtered through Durapore Membrane Filter 0.45 um HV (manufactured by Merck Millipore, registered trademark (the same holds true for the description below)), and then desalted and concentrated to obtain an enzyme extract.

This enzyme extract was applied to HiTrap Q HP column (manufactured by Amersham Biosciences Corp., registered trademark (the same holds true for the description below)) equilibrated with a 20 mM acetate buffer solution (pH 6.0) containing 0.05% sodium cholate and 0.1% Triton X-100. Then, elution was performed by the gradient elution method from the 20 mM acetate buffer solution (pH 6.0) containing 0.05% sodium cholate and 0.1% Triton X-100 into a buffer solution containing 1 M sodium chloride in a 20 mM acetate buffer solution (pH 6.0) containing 0.05% sodium cholate and 0.1% Triton X-100. Next, a fraction with strongly observed glucocerebrosidase activity was pooled, and desalted and concentrated by ultrafiltration. This desalted and concentrated solution was applied to HiTrap SP HP column (manufactured by Amersham Biosciences Corp.) equilibrated with a 20 mM acetate buffer solution (pH 5.5) containing 0.05% sodium cholate and 0.1% Triton X-100. Then, elution was performed for fractionation by the gradient elution method from the 20 mM acetate buffer solution (pH 5.5) containing 0.05% sodium cholate and 0.1% Triton X-100 into a buffer solution containing 1 M sodium chloride in a 20 mM acetate buffer solution (pH 5.78) containing 0.05% sodium cholate and 0.1% Triton X-100. Then, an enzyme fraction with strongly observed glucocerebrosidase activity was pooled, and desalted and concentrated by ultrafiltration. The resulting solution was used as rice-derived glucocerebrosidase (RGC1).

The glucocerebrosidase activity of the fraction was measured as follows: a predetermined amount of sample was added to a 50 mM acetate buffer solution (pH 5.5) containing 100 µM glucosylceramide (cerebroside B), 0.1% Tween 20, and 0.05% sodium cholate, and incubated at 37°C for 15 minutes. Subsequently, a 4-fold amount of ethanol was added to the obtained reaction solution, mixed therewith, and then centrifuged at 15000 rpm for 20 minutes. The centrifuged supernatant was subjected to high-performance liquid chromatography analysis. In the high-performance liquid chromatography analysis, the sample was injected to TSKgel ODS-120T column (4.6 mm × 30 cm), and a solvent having an ethanol concentration of 83% was flowed at a flow rate of 0.8 mL/min, followed by detection in a UV detector (ultraviolet absorption wavelength: 215 nm) to thereby measure the amount of ceramide newly generated through enzymatic reaction. The amount of ceramide was determined by using, as a standard, ceramide generated from cerebroside B by imiglucerase (manufactured by Sanofi S.A.), human-derived glucocerebrosidase. The molecular weight of the generated ceramide was examined by negative LC-MS (manufactured by Agilent Technologies, Inc.) to thereby confirm that it was ceramide generated from cerebroside B (ESI-MS m/z: 564.5[M-H]⁻). Glucocerebrosidase activity per mL of the enzyme solution was calculated when the amount of the enzyme that generated 1 pmol of ceramide for 1 minute in the enzymatic reaction solution was defined as 1 unit (U).

The protein concentration of the purified RGC1 was determined in Protein Assay Kit (manufactured by Bio-Rad Laboratories, Inc.) by using bovine serum albumin as a standard and imiglucerase whose protein concentration was measured in advance as a preparation. Specifically, the purified RGC1 solution and the imiglucerase solution were each injected to TSKgel Octyl-80Ts column (4.6 mm × 15 cm, manufactured by Tosoh Corp.), and solutions of acetonitrile in 0.05% trifluoroacetic acid were flowed in a gradient mode of elevated acetonitrile ratios at a flow rate of 0.8 mL/min, followed by detection in a UV detector (ultraviolet absorption wavelength: 280 nm). The resulting peak area was determined and compared with imiglucerase to thereby determine the protein concentration of the purified RGC1.

The fraction containing this RGC1 exhibited a single band in SDS-PAGE, and its average molecular weight (MW) was approximately 62 kD. The SDS-PAGE was performed using AE-6000 electrophoresis (manufactured by ATTO Corp.) and Precast Minigel e-PAGEL (E-R10L/gel concentration of 10%/18 samples, manufactured by ATTO Corp.). Silver staining was performed with Silver Stain MS Kit (manufactured by FUJIFILM Wako Pure Chemical Corp.). The molecular weight marker used was SDS-PAGE Molecular Weight Standards Low Range (manufactured by Bio-Rad Laboratories, Inc.).

### (Example 3)

RGC1 obtained in Example 2 was added to 12.5 µM glucosylceramide (one of the 12 animal-derived, plant-derived, or filamentous fungus-derived ones in Table 2 below) or synthetic β-glucoside, and a 50 mM acetate buffer solution (pH 5.5) containing 0.1% Tween 20 and 0.05% sodium cholate, and incubated at 37°C for 15 minutes. Subsequently, a 4-fold amount of ethanol was added to the obtained reaction solution, mixed therewith, and then centrifuged under a condition of 15000 rpm for 20 minutes. The amount of ceramide in the supernatant was measured by high-performance liquid chromatography using various reverse-phase columns to thereby determine glucocerebrosidase activity. The amount of ceramide was determined by using, as a standard, ceramide generated from the glucosylceramide by imiglucerase. The glucocerebrosidase activity was determined as relative activity when activity in the case of using animal-derived glucosylceramide d18:1(4*E*)-C8:0-GluCer as a substrate was defined as 100 (rightmost column of Table 2 below). This test was conducted five times, and average relative activity was determined therefrom. The results are shown in Table 2 below.

**[Table 2]**

| **Type of substrate** | **Sugar** | **Fatty acid** | **Sphingosine base** | **Relative activity (%)** |
|---|---|---|---|---|
| **Animal-derived** | | | | |
| **d18:1(4*E*)-C18:0-GM₃** | **NeuAc-Gal-Glu** | **18:0** | **d18:1(4*E*)** | **ND** |
| **d18:1(4*E*)-C8:0-LacCer** | **Lac** | **8:0** | **d18:1(4*E*)** | **3.4** |
| **d18:1(4*E*)-C8:0-GalCer** | **Gal** | **8:0** | **d18:1(4*E*)** | **5.8** |
| **d18:1(4*E*)-C8:0-GluCer** | **Glu** | **8:0** | **d18:1(4*E*)** | **100** |
| **d18:1(4*E*)-C12:0-GluCer** | **Glu** | **12:0** | **d18:1(4*E*)** | **82.9** |
| **d18:1(4*E*)-C18:0-GluCer** | **Glu** | **18:0** | **d18:1(4*E*)** | **50.4** |

| **Plant-derived** | | | | |
|---|---|---|---|---|
| **d18:2(4*E*,8*Z*)-C16h:0-GluCer** | **Glu** | **16h:0** | **d18:2(4*E*,8*Z*)** | **147.7** |
| **d18:2(4*E*,8*E*)-C16h:0-GluCer** | **Glu** | **16h:0** | **d18:2(4*E*,8*E*)** | **96.1** |
| **d18:2(4*E*,8Z)-C18h:0-GluCer** | **Glu** | **18h:0** | **d18:2(4*E*,8*Z*)** | **143.3** |

| **Filamentous fungus-derived** | | | | |
|---|---|---|---|---|
| **d19:2(4*E*,8*E*,9Me)-C16h:0-GluCer (cerebroside B)** | **Glu** | **16h:0** | **d19:2(4*E*,8*E*,9Me)** | **116.7** |
| **d19:2(4*E*,8*E*,9Me)-C16h:1-GluCer** | **Glu** | **16h:1** | **d19:2(4*E*,8*E*,9Me)** | **117.7** |
| **d19:2(4*E*,8*E*,9Me)-C18h:1-GluCer (cerebroside C)** | **Glu** | **18h:1** | **d19:2(4*E*,8*E*,9Me)** | **113.2** |

| **Synthetic β-glucoside** | | | | |
|---|---|---|---|---|
| ***p*NP-β-glucoside** | **Glu** | **-** | **-** | **2.5** |

| | | | | |
|---|---|---|---|---|
| Glu, glucose; Lac, lactose; Gal, galactose; NeuAc, acetylneuraminic acid; Cer, ceramide; GluCer, glucosylceramide; GalCer, galactosylceramide; LacGer, lactosylceramide; 16:0, palmitic acid; 16:1, (*E*)-3-hexadecenoic acid; 16h:1, 2-hydroxy-(*E*)-3-hexadecenoic acid; 18:0, stearic acid; 18:1, (*E*)-3-octadecenoic acid;18h:1, 2-hydroxy-(*E*)-3-octadecenoic acid; d18:1(4*E*), (*E*)-4-sphingenine; d18:2(4*E*,8*E*), (4*E*,8*E*)-4,8-sphingadienine; d19:2(4*E*,8*E*,9Me), (4*E*,8*E*)-9-methyl-4,8-sphingadienine; *p*NP-β-glucoside, 4-nitrophenyl-β-D-glucopyranoside | | | | |

As a result, RGC1 rarely reacted when lactosylceramide such as d18:1(4*E*)-C18:0-GM₃ or d18:1(4*E*)-C8:0-LacCer and galactosylceramide such as d18:1(4*E*)-C8:0-GalCer were used as substrates, revealing that it reacted by specifically recognizing the glucose structure of glucosylceramide. Further, RGC1 also rarely reacted with *p*NP-β-glucoside, revealing that it reacted by specifically recognizing the ceramide structure of glucosylceramide. From these results, RGC1 was found to be glucocerebrosidase. It was also shown that RGC1 also reacted with any type of glucosylceramide substrate derived from a plant, an animal, or a filamentous fungus.

### (Example 4)

Four types, glucocerebrosidase 1 (GBA1; imiglucerase, belonging to GH30), glucocerebrosidase 2 (GBA2, belonging to GH116), glucocerebrosidase 3 (GBA3, belonging to GH1), and lactase-phlorizin hydrolase (belonging to GH1), are known as animal-derived glucocerebrosidase. Glucocerebrosidase belonging to GH116 (AtGCD3) has been isolated as plant-derived glucocerebrosidase from thale-cress. Accordingly, the *K*_{cat}/*K*ₘ values (values obtained by dividing the number of molecules of a substrate that can be converted for 1 second by one molecule of an enzyme by a substrate concentration that provides a reaction rate of 50% of the maximum reaction rate of the enzyme) of RGC1 obtained in Example 2 and imiglucerase were determined by the Hanes-Woolf plot in accordance with the reaction method of Example 2 using a synthetic glucosylceramide substrate *N*-[6-[(7-nitro-2-1,3-benzoxadiazol-4-yl)amino]hexanoyl]-D-glucosyl-β1-1'-sphingosine (C6-NBD glucosylceramide) . The *K*_{cat}/*K*ₘ values of animal-derived glucocerebrosidase GBA3 and thale-cress-derived glucocerebrosidase AtGCD3 were taken from documented values (Journal of Biological Chemistry 282, (2007) 30889-30900, and Journal of Biological Chemistry 295, (2020) 717-728). The results are shown in Table 3 below. From the results, RGC1 had an evidently high *K*_{cat}/*K*ₘ value compared with other glucocerebrosidases, revealing that it was excellent glucocerebrosidase.

**[Table 3]**

| | ***K*_{cat}/*K*ₘ(s⁻¹/µM)** |
|---|---|
| **RGC1** | **1.97** |
| **Imiglucerase (GBA1)** | **0.419** |
| **GBA3** | **0.0262** |
| **AtGCD3** | **0.0053** |

### (Example 5)

RGC1 obtained in Example 2 and imiglucerase were each added to a 50 mM acetate buffer solution (pH 5.5) containing 100 µM glucosylceramide (cerebroside B), 0.1% Tween 20, and 0.05% sodium cholate, and incubated at each temperature for 15 minutes. Subsequently, a 4-fold amount of ethanol was added to the obtained reaction solution, mixed therewith, and then centrifuged under a condition of 15000 rpm for 20 minutes. The supernatant was subjected to high-performance liquid chromatography analysis to measure the amount of ceramide generated through enzymatic reaction. The high-performance liquid chromatography analysis was carried out in the same manner as in Example 2. The amount of ceramide and enzymatic activity were also calculated in the same manner as in Example 2. A temperature that attained the highest glucocerebrosidase activity was regarded as the optimum temperature. This test was conducted three times, and an average value was determined therefrom. The results are shown in Table 4 below.

From the results, the optimum temperature of the human-derived glucocerebrosidase imiglucerase was 42.5°C, whereas the optimum temperature of the rice-derived glucocerebrosidase RGC1 was 54.0°C, suggesting that RGC1 was highly stable *in vivo.*

**[Table 4]**

| | **Optimum temperature (°C)** |
|---|---|
| **Imiglucerase** | **42.5** |
| **RGC1** | **54.0** |

### (Example 6)

For enzymes, it is generally known that a higher optimum temperature leads to better thermostability. From Example 5, RGC1 was expected to have thermostability because its optimum temperature for glucocerebrosidase activity was higher than that of imiglucerase. Accordingly, the following test was carried out in order to confirm thermostability in lysosome (pH 5) and cytoplasm (pH 7) where glucocerebrosidase typically resides and acts *in vivo* in humans.

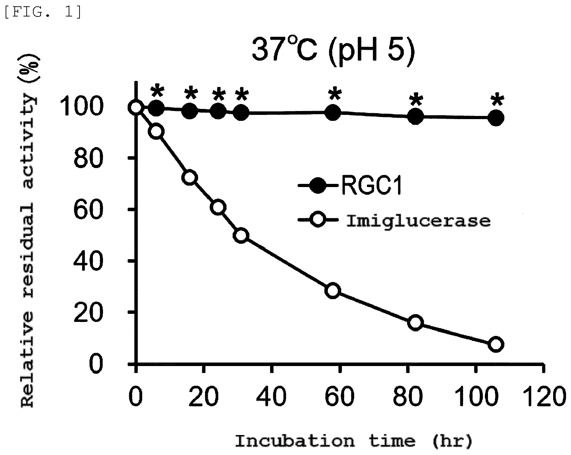

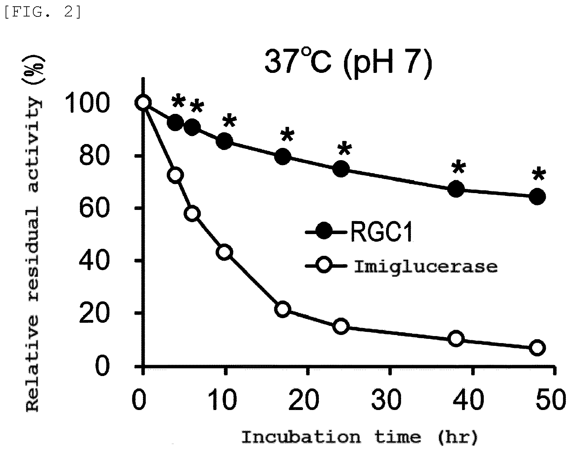

RGC1 obtained in Example 2 and imiglucerase were each incubated at pH 5 simulating lysosome (50 mM acetate buffer solution (pH 5.0) containing 0.1% Triton X-100 and 0.05% sodium cholate) at 37°C for 6 to 106 hours or at pH 7 simulating cytoplasm (50 mM potassium phosphate buffer solution (pH 7.0) containing 0.1% Triton X-100 and 0.05% sodium cholate) at 37°C for 4 to 48 hours. Glucocerebrosidase activity after the incubation for each time was measured in accordance with the method of Example 2. Residual activity was calculated as a relative activity value when activity before incubation was defined as 100. This test was conducted three times, and an average value was determined therefrom. The results are shown in Figs. 1 and 2. In these figures, the mark * was added when the glucocerebrosidase activity of RGC1 thus reacted for each time was significantly higher than that of imiglucerase (1% significance).

From Figs. 1 and 2, RGC1 was evidently superior in stability to imiglucerase at both pH 5 and pH 7, indicating that it had thermostability.

### (Example 7)

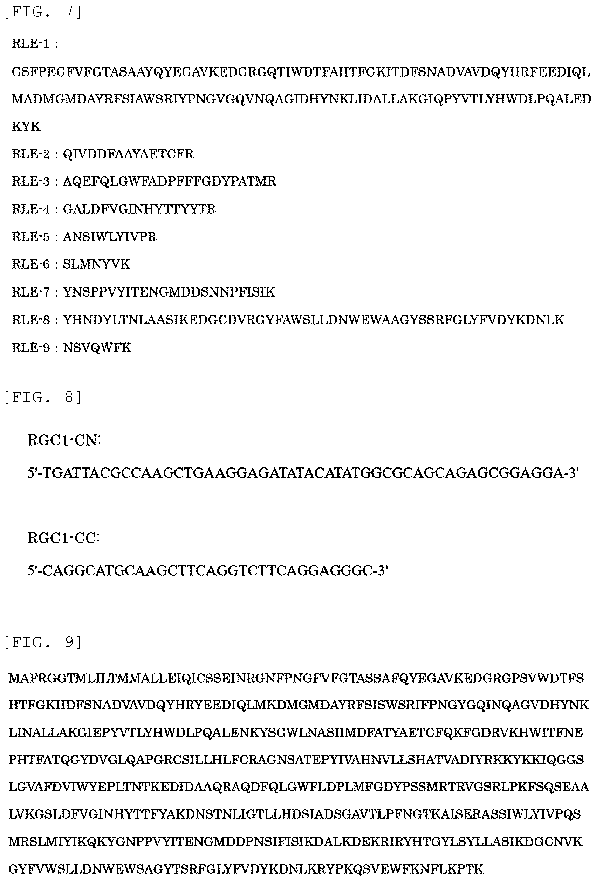

The RGC1 band obtained by SDS-PAGE in Example 2 was excised and then treated with trypsin. The molecular masses of the obtained peptide fragments were analyzed with a matrix assisted laser desorption/ionization time-of-flight mass spectrometer (MALDI-TOFMS: Microflex LRF 20, manufactured by Bruker Daltonics) to determine the amino acid sequences of the 9 peptide fragments shown in SEQ ID NOs: 5 to 13 in the sequence listing and Fig. 7.

The precise molecular weights of these 9 peptide fragments of RGC1 were completely consistent with the precise molecular weights of 9 peptides derived from a rice (*Oryza sativa* L. cv. Nipponbare)-derived protein called Os3BGlu6 with an unidentified function, suggesting the possibility that RGC1 was a protein identical to Os3BGlu6. It should be noted that there has been no report so far stating that Os3BGlu6 is glucocerebrosidase.

### (Example 8)

Total RNA was extracted from the callus of rice (*Oryza sativa* L. cv. Nipponbare) using RNeasy Plant Mini Kit (manufactured by Qiagen N.V., registered trademark (the same holds true for the description below)). mRNA was purified from the total RNA using Absolutely mRNA Purification Kit (manufactured by Agilent Technologies, Inc.). cDNA synthesis from the mRNA and subsequent RACE analysis were performed using SuperScript III Reverse Transcriptase (manufactured by Thermo Fisher Scientific, Inc., registered trademark). Os3BGlu6 cDNA was obtained by PCR amplification using the two primers (F-primer (RGC1-CN), and R-primer (RGC1-CC)) shown in SEQ ID NOs: 14 and 15 in the sequence listing and Fig. 8 which were prepared from an open reading frame presumed from the whole genomic sequence of rice *(Oryza sativa* L. cv. Nipponbare).

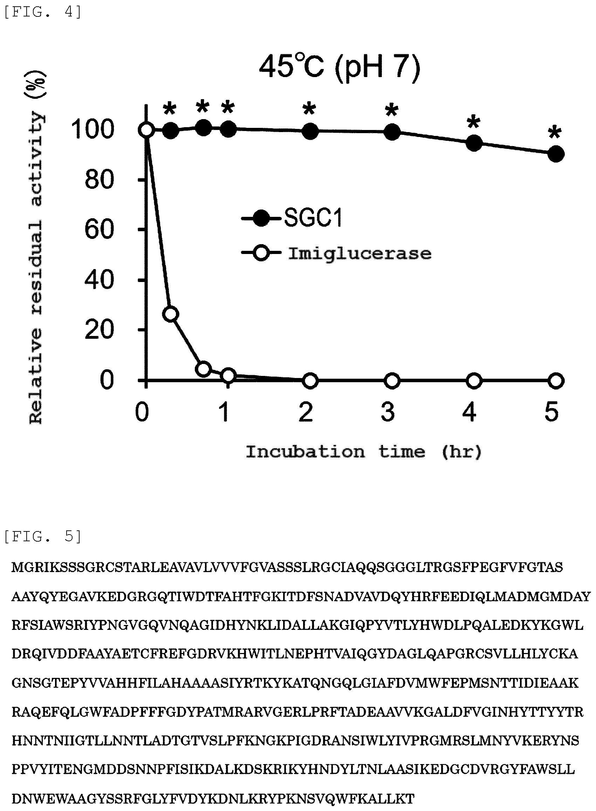

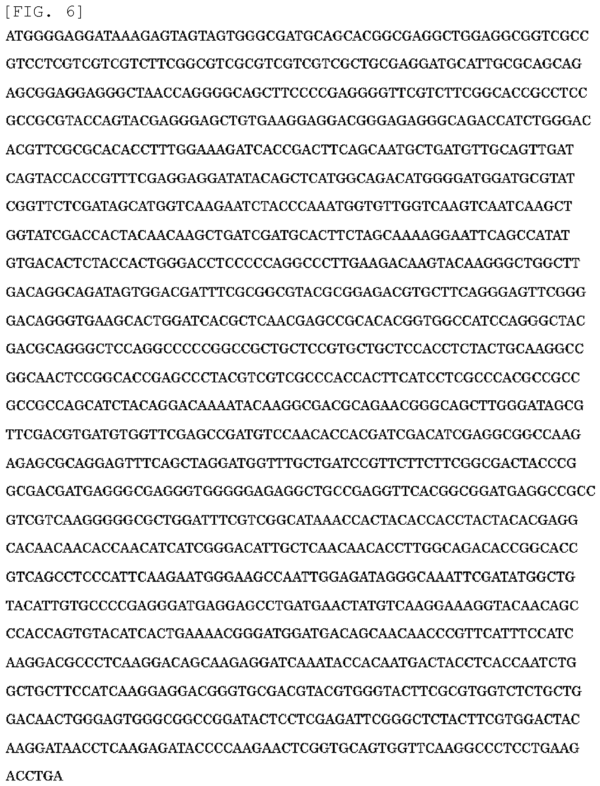

Specific conditions for PCR involved adding KOD-Plus-Neo (manufactured by Toyobo Co., Ltd.) to the rice callus cDNA and the two primers, and repeating reactions conditions of 94°C for 2 minutes, 60°C for 0.5 minutes, and 68°C for 1 minute 35 times for amplification. Then, the amplified fragment was subcloned into a plasmid vector pUC19. Further, a longer DNA region also including DNA regions upstream and downstream of the amplified fragment was amplified by a similar method, and the nucleotide sequence of the fragment was analyzed by a standard method to thereby determine the whole nucleotide sequence of cDNA of the Os3BGlu6 gene. This nucleotide sequence is shown in SEQ ID NO: 3 in the sequence listing and Fig. 6. An amino acid sequence translated from this nucleotide sequence is shown in SEQ ID NO: 1 in the sequence listing and Fig. 5. This amino acid sequence revealed that Os3BGlu6 belonged to GH1.

### (Example 9)

The presence or absence of glucocerebrosidase activity in Os3BGlu6 produced in *Escherichia coli* was examined in order to reveal that the isolated Os3BGlu6 gene was glucocerebrosidase gene (gene encoding RGC1). pUC19 obtained in Example 8 in which the Os3BGlu6 gene was subcloned was used. *Escherichia coli* (DH5α) was transformed by transfer thereof. Next, this transformant was cultured at 37°C for 24 hours in LB liquid medium (1.0% tryptone, 0.5% yeast extracts, 1.0% sodium chloride, 50 ug/mL ampicillin) and centrifuged under a condition of 15000 rpm for 10 minutes to harvest the bacterial cells. The obtained bacterial cells were washed twice with a 50 mM acetate buffer solution (pH 6.0) containing 0.05% sodium cholate. Then, centrifugation was performed under a condition of 15000 rpm for 10 minutes to harvest the bacterial cells. The obtained bacterial cells were suspended in a 50 mM acetate buffer solution (pH 6.0) containing 0.3% Triton X-100 and 0.05% sodium cholate, followed by sonication. This sonicated homogenate was centrifuged for 60 minutes under conditions of 18000 rpm and 4°C. Then, the centrifuged supernatant was filtered through Durapore Membrane Filter 0.45 um HV (manufactured by Merck Millipore, registered trademark (the same holds true for the description below)), and then desalted and concentrated to obtain an enzyme extract. This enzyme extract was added to a 50 mM sodium acetate buffer solution (pH 5.5) containing 100 µM glucosylceramide (cerebroside C), 0.1% Tween 20, and 0.05% sodium cholate, and incubated at 37°C for 13 minutes. Subsequently, a 4-fold amount of ethanol was added to the obtained reaction solution, mixed therewith, and then centrifuged under a condition of 15000 rpm for 20 minutes. The supernatant was subjected to high-performance liquid chromatography analysis to measure the amount of ceramide generated through enzymatic reaction. The high-performance liquid chromatography analysis was carried out in the same manner as in Example 1. The molecular weight of the generated ceramide was examined by negative LC-MS (manufactured by Agilent Technologies, Inc.) to thereby confirm that it was ceramide generated from cerebroside C (ESI-MS m/z: 590.5[M-H]⁻). Glucocerebrosidase activity per mL of the enzyme solution was calculated when the amount of the enzyme that generated 1 µmol of ceramide for 1 minute in the enzymatic reaction solution was defined as 1 unit (U). The results are shown in Table 5 below.

**[Table 5]**

| | **Glucocerebrosidase activity (U/mL)** |
|---|---|
| ***Escherichia coli* transformed with Os3BGlu6 gene** | **0.180** |
| ***Escherichia coli* transformed with only plasmid** | **0.000** |

The results indicated that the enzyme extract obtained from *Escherichia coli* transformed with the Os3BGlu6 gene evidently had glucocerebrosidase activity. This revealed that the isolated RGC1 was Os3BGlu6 and was glucocerebrosidase belonging to GH1. It should be noted that there has been no report so far stating that glucocerebrosidase belonging to GH1 has been found from plants.

### (Example 10)

The homology (identity) between the amino acid sequence of RGC1 and the amino acid sequences of animal-derived glucocerebrosidase or thale-cress-derived glucocerebrosidase AtGCD3 was examined using EMBOSS Needle (https://www.ebi.ac.uk/Tools/psa/emboss_needle/). As a result, the homology was 14.9% to GBA1, 10.1% to GBA2, 35.8% to GBA3, 11.3% to lactase-phlorizin hydrolase, and 3.3% to AtGCD3. This result revealed that RGC1 was glucocerebrosidase that was also totally different in amino acid sequence from the previously isolated glucocerebrosidase.

### (Example 11)

102 g of stems of soybean (*Glycine max* (L.) Merr. Enrei) was collected, chopped, and then homogenized with a homogenizer in a 20 mM acetate buffer solution (pH 5.5) containing 0.01% sodium cholate. This homogenate was centrifuged for 60 minutes under conditions of 10000 rpm and 4°C. The centrifuged supernatant was removed. Further, a 20 mM acetate buffer solution (pH 5.5) containing 0.01% sodium cholate was added to the centrifuged precipitate, stirred for 5 minutes, and then centrifuged for 60 minutes under conditions of 10000 rpm and 4°C. The centrifuged supernatant was removed. Further, an enzyme extract was obtained from the centrifuged precipitate in the same manner as in Example 2.

This enzyme extract was applied to HiTrap Q HP column (manufactured by Amersham Biosciences Corp.) equilibrated with a 0.5 mM phosphate buffer solution (pH 6.7) containing 0.05% sodium cholate and 0.05% Triton X-100. Then, elution was performed by the gradient elution method from the 0.5 mM phosphate buffer solution (pH 6.7) containing 0.05% sodium cholate and 0.05% Triton X-100 into a solution containing 1 M sodium chloride in a 20 mM phosphate buffer solution (pH 6.7) containing 0.05% sodium cholate and 0.1% Triton X-100. Next, a fraction with strongly observed glucocerebrosidase activity was pooled, and desalted and concentrated by ultrafiltration. This desalted and concentrated solution was applied to HiTrap SP HP column (manufactured by Amersham Biosciences Corp.) equilibrated with a 0.5 mM acetate buffer solution (pH 6.0) containing 0.05% sodium cholate and 0.05% Triton X-100. Then, elution was performed for fractionation by the gradient elution method from the 0.5 mM acetate buffer solution (pH 6.0) containing 0.05% sodium cholate and 0.05% Triton X-100 into a solution containing 1 M sodium chloride in a 0.5 mM acetate buffer solution (pH 6.0) containing 0.05% sodium cholate and 0.05% Triton X-100. As a result, a fraction with strongly observed glucocerebrosidase activity was pooled, and desalted and concentrated by ultrafiltration. The resulting solution was used as soybean-derived glucocerebrosidase (SGC1).

The glucocerebrosidase activity of the fraction and the protein concentration of SGC1 were analyzed by the same methods as in Example 2. This SGC1 fraction exhibited a single band in SDS-PAGE, and its average molecular weight (MW) was approximately 62.0 kD. The SDS-PAGE was also performed by the same method as in Example 2.

### (Example 12)

The optimum temperatures for the glucocerebrosidase activity of SGC1 obtained in Example 11 and imiglucerase were confirmed by the same method as in Example 5. The results are shown in Table 6 below.

From the results, the optimum temperature of imiglucerase was 42.5°C, whereas the optimum temperature of SGC1 was very high, namely 64.0°C, suggesting the possibility that SGC1 was highly stable *in vivo.*

**[Table 6]**

| | **Optimum temperature (°C)** |
|---|---|
| **Imiglucerase** | **42.5** |
| **SGC1** | **64.0** |

### (Example 13)

For enzymes, as mentioned above, it is known that a higher optimum temperature leads to better thermostability. From Example 12, SGC1 was expected to have thermostability because its optimum temperature for glucocerebrosidase activity was higher than that of imiglucerase. Accordingly, the following test was carried out in order to confirm thermostability in lysosome (pH 5) and cytoplasm (pH 7).

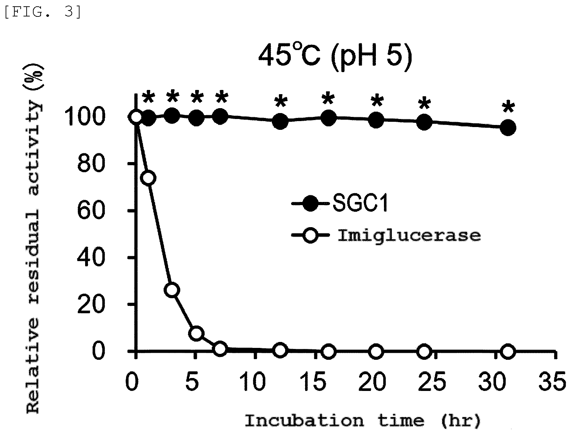

Two glucocerebrosidases, SGC1 obtained in Example 11 and imiglucerase, were each incubated at pH 5 simulating lysosome (50 mM acetate buffer solution (pH 5.0) containing 0.1% Triton X-100 and 0.05% sodium cholate) at 45°C for 1 to 31 hours or at pH 7 simulating cytoplasm (50 mM potassium phosphate buffer solution (pH 7.0) containing 0.1% Triton X-100 and 0.05% sodium cholate) at 45°C for 0.3 to 5 hours. Glucocerebrosidase activity after the reaction for each time was measured in accordance with the method of Example 2. Residual activity was calculated as a relative activity value when activity before incubation was defined as 100. This test was conducted three times, and an average value was determined therefrom. The results are shown in Figs. 3 and 4. In these figures, the mark * was added when the glucocerebrosidase activity of SGC1 thus reacted for each time was significantly higher than that of imiglucerase (1% significance).

From Figs. 3 and 4, SGC1 was evidently much superior in stability to imiglucerase at both pH 5 and pH 7, indicating that it had thermostability.

### (Example 14)

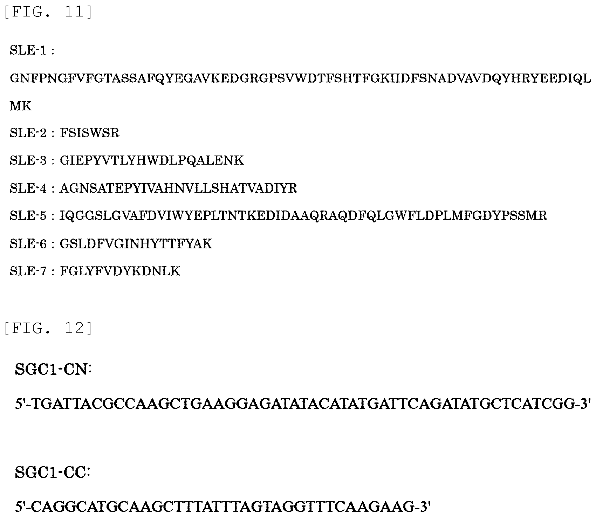

The SGC1 band obtained by SDS-PAGE in Example 11 was excised and then treated with trypsin. The molecular masses of the obtained peptide fragments were analyzed with a matrix assisted laser desorption/ionization time-of-flight mass spectrometer (MALDI-TOFMS: Microflex LRF 20, manufactured by Bruker Daltonics) to determine the amino acid sequences of the 7 peptide fragments shown in SEQ ID NOs: 16 to 22 in the sequence listing and Fig. 11.

The precise molecular weights of these 7 peptide fragments derived from SGC1 were completely consistent with the precise molecular weights of 7 peptides derived from a U.S. soybean cultivar *(Glycine max* (L.) Merr. Williams 82)-derived protein called β-glucosidase 40 with an unidentified function, suggesting the possibility that SGC1 was a protein identical to β-glucosidase 40. It should be noted that this β-glucosidase 40 has been simply designated from homology search with genomic sequences and has not been confirmed to have enzymatic activity as β-glucosidase; and furthermore, there has been no report so far stating that it is glucocerebrosidase.

### (Example 15)

Total RNA was extracted from the leaves of soybean (*Glycine max* (L.) Merr. Enrei) using RNeasy Plant Mini Kit (manufactured by Qiagen N.V.). cDNA was synthesized from this total RNA using PrimeScript II 1st strand cDNA Synthesis Kit (manufactured by Takara Bio Inc.). cDNA of β-glucosidase 40 derived from soybean (*Glycine max* (L.) Merr. Enrei) was obtained by PCR amplification using the two primers (F-primer (SGC1-CN) and R-primer (SGC1-CC)) shown in SEQ ID NOs: 23 and 24 in the sequence listing and Fig. 12 which were prepared from an open reading frame presumed from the whole genomic sequence of the U.S. soybean cultivar (*Glycine max* (L.) Merr. Williams 82).

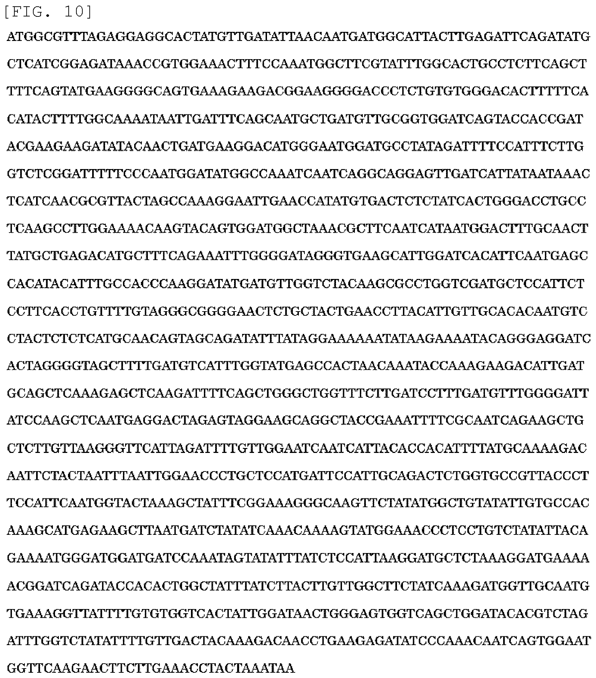

Specific conditions for PCR involved adding KOD-Plus-Neo (manufactured by Toyobo Co., Ltd.) to the cDNA from the leaves of soybean *(Glycine max* (L.) Merr. Enrei) and the two primers, and repeating reaction conditions of 94°C for 2 minutes, 60°C for 0.5 minutes, and 68°C for 1 minute 40 times for amplification. Then, the amplified fragment was subcloned into a plasmid vector pUC19. Further, a longer DNA region also including DNA regions upstream and downstream of the amplified fragment was amplified by a similar method, and the nucleotide sequence of the fragment was analyzed by a standard method to thereby determine the whole nucleotide sequence of cDNA of the β-glucosidase 40 gene derived from soybean (*Glycine max* (L.) Merr. Enrei). This nucleotide sequence is shown in SEQ ID NO: 4 in the sequence listing and Fig. 10. An amino acid sequence translated from this nucleotide sequence is shown in SEQ ID NO: 2 in the sequence listing and Fig. 9. This amino acid sequence revealed that β-glucosidase 40 derived from soybean (*Glycine max* (L.) Merr. Enrei) belonged to GH1.

### (Example 16)

The presence or absence of glucocerebrosidase activity in soybean-derived β-glucosidase 40 produced in *Escherichia coli* was examined in order to reveal that the β-glucosidase 40 gene derived from soybean (*Glycine max* (L.) Merr. Enrei) was glucocerebrosidase gene. pUC19 obtained in Example 15 in which the soybean-derived β-glucosidase 40 gene was subcloned was used. *Escherichia coli* (DH5α) was transformed by transfer thereof. Glucocerebrosidase activity per mL of an enzyme extract was calculated by the same method as in Example 9 except that the enzymatic reaction was incubated at 60°C for 30 minutes using this transformant. The results are shown in Table 7 below.

**[Table 7]**

| | **Glucocerebrosidase activity (U/mL)** |
|---|---|
| ***Escherichia coli* transformed with soybean-derived β-glucosidase 40 gene** | **0.0123** |
| ***Escherichia coli* transformed with only plasmid** | **0.0000** |

The results indicated that the enzyme extract obtained from *Escherichia coli* transformed with the β-glucosidase 40 gene derived from soybean (*Glycine max* (L.) Merr. Enrei) evidently had glucocerebrosidase activity. This revealed that the isolated soybean-derived SGC1 was soybean-derived β-glucosidase 40 and was glucocerebrosidase belonging to GH1. As mentioned above, it should be noted that there has been no report so far stating that glucocerebrosidase belonging to GH1 has been found from plants.

### (Example 17)

The homology (identity) between the amino acid sequence of SGC1 and the amino acid sequences of animalderived glucocerebrosidase or thale-cress-derived glucocerebrosidase AtGCD3 was examined using EMBOSS Needle described above. As a result, the homology was 14.9% to GBA1, 10.0% to GBA2, 35.9% to GBA3, 10.4% to lactase-phlorizin hydrolase, and 7.6% to AtGCD3. This result revealed that SGC1 was glucocerebrosidase that was also totally different in amino acid sequence from the previously isolated glucocerebrosidase.

### (Example 18)

A protein having high homology to the amino acid sequence of RGC1 was searched for by use of BLASTp search (https://blast.ncbi.nlm.nih.gov/Blast.cgi). As a result, it was revealed that a wide range of seed plants had highly homologous proteins. The homology (identity) was examined using EMBOSS Needle described above. The results are shown in Table 8 below.

**[Table 8]**

| **Accession No. and protein name** | **Origin** | **Homology** (%) |
|---|---|---|
| **Monocotyledonous plant** | | |
| XP_015613193.1 beta-glucosidase 34 | Rice (Oryza sativa Japonica Group) | 77.4% |
| VAI08420.1 unnamed protein product | Wheat (Triticum turgidum subsp. durum) | 84.4% |
| KAE8788864.1 Beta-glucosidase 34 | Barley (Hordeum vulgare) | 73.8% |
| PWZ58470.1 Beta-glucosidase 6 | Maize (Zea mays) | 84.2% |
| XP_002465652.2 beta-glucosidase 6 | Sorghum (Sorghum bicolor) | 82.7% |
| RLN17293.1 beta-glucosidase 6 | Proso millet (Panicum miliaceum) | 83.7% |
| XP_004985215.1 beta-glucosidase 6 | Foxtail millet (Setaria italica) | 84.9% |
| **Dicotyledonous plant** | | |
| NP_173978.1 beta glucosidase 40 | Arabidopsis thaliana | 68.8% |
| XP_013642074.1 beta-glucosidase 40-like | Rapeseed (Brassica napus) | 69.5% |
| XP_013587431.1 beta-glucosidase 40 | Cabbage (Brassica oleracea var. oleracea) | 69.5% |
| XP_011657400.1 PREDICTED:beta-glucosidase 40 | Cucumber (Cucumis sativus) | 68.1% |
| XP_008445465.1 PREDICTED:beta-glucosidase 40 | Melon (Cucumis melo) | 67.9% |
| XP_004228406.1 beta-glucosidase 40 | Tomato (Solanum lycopersicum) | 63.6% |
| XP_016497770.1 beta-glucosidase 40-like | Tobacco (Nicotiana tabacum) | 64.6% |
| XP_006365136.1 beta-glucosidase 40-like | Potato (Solanum tuberosum) | 64.2% |
| XP_003556662.1 beta-glucosidase 40 SGC1 | Soybean (Glycine max (L.) Merr. Enrei) | 67.6% |
| XP_019445904.1 beta-glucosidase 40-like | Lupine (Lupinus angustifolius) | 67.0% |
| PRQ40673.1 putative beta-glucosidase | Rosa chinensis | 62.6% |
| PQQ20207.1 beta-glucosidase 40 | Cherry (Prunus yedoensis var. nudiflora) | 65.0% |
| WA37410.1 PREDICTED:beta-glucosidase | Almond (Prunus dulcis) | 69.4% |
| XP_023754232.1 beta-glucosidase 40 | Lettuce (Lactuca sativa) | 67.0% |
| XP_017616432.1 PREDICTED:beta-glucosidase 40 | Cotton plant (Gossypium arboreum) | 68.8% |
| KAE8732915.1 Beta-glucosidase 34 | Hibiscus (Hibiscus syriacus) | 65.8% |
| XP_017224511.1 beta-glucosidase 6-like | Carrot (Daucus carota subsp. sativus) | 70.0% |

| **Fern** | | |
|---|---|---|
| XP_002967091.1 beta-glucosidase 40 | Selaginella moellendorffii | 53.2% |

| **Liverwort** | | |
|---|---|---|
| PTQ42983.1 hypothetical protein | Marchantia polymorpha | 46.6% |
| XP_024362173.1 beta-glucosidase 6-like | Physcomitrella patens | 51.4% |

From Table 8, the amino acid sequence of RGC1 exhibited homology of 73.8 to 84.9% to β-glucosidase 6 and β-glucosidase 34 derived from monocotyledonous plants, and exhibited homology of 62.6 to 70.0% to β-glucosidase 6, β-glucosidase 40 and β-glucosidase 34 derived from dicotyledonous plants. It also exhibited homology of 46.6 to 53.2% to fern or liverwort β-glucosidase 6 or β-glucosidase 40. All of these amino acid sequences had a conservative region necessary for GH1 and belonged to GH1. From this result, the β-glucosidase 6, the β-glucosidase 40 and the β-glucosidase 34 described above were considered glucocerebrosidase.

### (Example 19)

The following test was carried out in order to examine the presence or absence of similar glucocerebrosidase activity in plants other than rice. 0.01 to 1 g each of sites of leaves, roots, stems, petals, pistils, pollens, fruits, rhizoids, and sporangia of various plants was collected and homogenized with a homogenizer in a 50 mM acetate buffer solution (pH 5.0) containing 0.05% sodium cholate and 0.3% Triton X-100. This homogenate was centrifuged for 20 minutes under conditions of 15000 rpm and 4°C. The centrifuged supernatant was used as an enzyme extract.

Then, this enzyme extract was incubated at 45°C for 60 minutes in a 50 mM acetate buffer solution (pH 5.0) containing 100 µM glucosylceramide (cerebroside B), 0.05% sodium cholate, and 0.2% Triton X-100. Subsequently, a 4-fold amount of ethanol was added to the obtained reaction solution, mixed therewith, and then centrifuged under a condition of 15000 rpm for 20 minutes. The supernatant was subjected to high-performance liquid chromatography analysis to measure the amount of ceramide generated through enzymatic reaction. The high-performance liquid chromatography analysis was carried out in the same manner as in Example 2. The amount of ceramide and enzymatic activity were also calculated in the same manner as in Example 2. The number of units of glucocerebrosidase activity per g of a plant raw weight was calculated. This test was conducted three times, and an average value was determined therefrom. The results are shown in Table 9 below.

**[Table 9]**

| | Glucocerebrosidase activity (U/g): - represents unmeasured | | | | | | | | |
|---|---|---|---|---|---|---|---|---|---|
| | Leaf | Stem | Root | Petal | Pistil | Pollen | Fruit | Rhizoid | Sporangium |
| Wheat | 0.000486 | 0.000985 | 0.000363 | - | 0.0196 | 0.12 | - | - | - |
| Rice | 0.0092 | 0.0196 | 0.057 | - | 0.07 | 0.94 | 0.0138 | - | - |
| Maize | 0.00105 | 0.0013 | 0.00172 | - | 0.00339 | 0.4 | 0.00344 | - | - |
| Tulip | 0.00031 | 0.00446 | 0.00186 | 0.00218 | 0.0104 | 0.253 | - | - | - |
| Lily | 0.00617 | 0.00724 | 0.00493 | 0.00226 | 0.00498 | 0.0389 | - | - | - |
| Goldenmane tickseed | 0.00189 | 0.00139 | 0.000966 | 0.027 | 0.00335 | 0.212 | - | - | - |
| Gazania | 0.000128 | 0.000159 | 0.000343 | 0.0113 | 0.0381 | 0.0443 | - | - | - |
| Gerbera | 0.000167 | 0.000135 | 0.000209 | 0.000755 | 0.0343 | 0.0112 | - | - | - |
| Rapeseed | 0.0134 | 0.0042 | 0.000489 | 0.0095 | 0.0284 | 0.33 | - | - | - |
| Arabidopsis thaliana | 0.0172 | 0.0422 | 0.000366 | 0.0109 | 0.0364 | 0.242 | 0.0185 | - | - |
| Cabbage | 0.0153 | 0.00898 | 0.000912 | 0.00471 | 0.025 | 0.183 | - | - | - |
| Melon | 0.00527 | 0.00278 | 0.00165 | 0.000335 | 0.000457 | 0.26 | 0.000022 | - | - |
| Cucumber | 0.000622 | 0.00161 | 0.00123 | 0.00116 | 0.00363 | 0.504 | 0.000547 | - | - |
| Broad bean | 0.0224 | 0.0142 | 0.00431 | 0.00671 | 0.00864 | 0.18 | 0.00621 | - | - |
| Soybean | 0.0111 | 0.0175 | 0.00516 | 0.00163 | 0.00265 | 0.117 | 0.00162 | - | - |
| Cotton plant | 0.00134 | 0.000548 | 0.00228 | 0.00313 | 0.000179 | 0.0237 | - | - | - |
| Hibiscus | 0.0143 | 0.00853 | 0.00366 | 0.00174 | 0.00208 | 0.105 | - | - | - |
| Cherry | 0.000028 | 0.000029 | 0.000157 | 0.000545 | 0.00021 | 0.152 | - | - | - |
| Rose | 0.000023 | 0.000096 | 0.000354 | 0.000663 | 0.0105 | 0.013 | - | - | - |
| Eggplant | 0.00233 | 0.00531 | 0.00107 | 0.0028 | 0.0193 | 0.74 | 0.00161 | - | - |
| Tomato | 0.000435 | 0.000106 | 0.000416 | 0.000819 | 0.00488 | 0.816 | 0.00128 | - | - |
| Tobacco | 0.00555 | 0.00191 | 0.00171 | 0.000973 | 0.0264 | 0.197 | - | - | - |
| Carrot | 0.002 | 0.00316 | 0.00183 | 0.0119 | 0.0154 | 0.449 | - | - | - |
| Japanese iris | 0.00314 | 0.00471 | 0.00342 | 0.00866 | 0.00366 | 0.0377 | - | - | - |
| Gingko | 0.000744 | 0.0198 | 0.00113 | - | 0.0000217 | 0.0296 | 0.000005 | - | - |
| Pine | 0.000772 | 0.00231 | 0.000368 | - | 0.000013 | 0.042 | - | - | - |
| Cryptomeria japonica | 0.0000953 | 0.0135 | 0.000399 | - | 0.0000403 | 0.46 | - | - | - |
| *Thelypteris acuminata* | 0.0000337 | 0.00000933 | 0.000013 | - | - | - | - | - | 0.000215 |
| Dryopteris crassirhizoma | 0.000432 | 0.000194 | 0.00052 | - | - | - | - | - | 0.000299 |
| Selaginella moellendorffii | 0.0000117 | 0.000091 | 0.000359 | - | - | - | - | - | 0.0000913 |
| Equisetum arvense | 0.0000857 | 0.000219 | 0.000294 | - | - | - | - | - | 0.00056 |
| Lygodium japonicum | 0.000013 | 0.000163 | 0.000016 | - | - | - | - | - | 0.000008 |
| *Pogonatum inflexum* | 0.000123 | 0.000408 | - | - | - | - | - | 0.000149 | - |
| *Pseudospiridentopsis horrida* | 0.000194 | 0.000334 | - | - | - | - | - | 0.000608 | - |
| *Hypnum plumaeforme* | 0.000068 | 0.0000297 | - | - | - | - | - | 0.000186 | - |
| *Pyrrhobryum dozyanum* | 0.00000733 | 0.0000563 | - | - | - | - | - | 0.000077 | - |
| *Campy*/*opus umbellatus* | 0.00000767 | 0.000122 | - | - | - | - | - | 0.0000613 | - |
| Dicranum japonicum | 0.000064 | 0.0000467 | - | - | - | - | - | 0.000313 | - |

From the results, glucocerebrosidase activity was observed in the measured extracts of all the plants. In consideration of the results of Tables 8 and 9, glucocerebrosidase was shown to exist in not only rice or soybean but all plants.

Furthermore, seed plants were shown to have glucocerebrosidase belonging to GH1 because monocotyledonous plant-derived RGC1 and dicotyledonous plant-derived SGC1 were glucocerebrosidase belonging to GH1 (Examples 9 and 16); enzymes that had high homology to the amino acid sequence of RGC1 and belonged to GH1 existed in many seed plants (Example 18); and as shown in Table 9 described above, high glucocerebrosidase activity was detected from extracts from many seed plants. Furthermore, this is enough to suggest that fern and liverwort also have glucocerebrosidase belonging to GH1 because β-glucosidase 6 or β-glucosidase 40 existed in fern or liverwort (Table 8 mentioned above); and glucocerebrosidase activity was also detected in extracts from fern or liverwort.

Next, homology will be discussed. Monocotyledonous plant-derived RGC1 and dicotyledonous plant-derived SGC1 demonstrated to be glucocerebrosidase belonging to GH1 in Examples 9 and 16 have homology of 67.6% between their amino acid sequences. It is therefore considered that an enzyme at least having homology equal to or more than 67.5% to the amino acid sequences of RGC1 and SGC1 is reliably glucocerebrosidase belonging to GH1. From Table 9 described above, seed plants were found to have glucocerebrosidase. It is therefore considered that an enzyme at least having homology equal to or more than 62.6% (China rose) which is the lowest homology to the amino acid sequence of RGC1 among the seed plants as shown in Table 8 mentioned above is also glucocerebrosidase belonging to GH1.

### (Example 20)

Enzyme extracts derived from various plants which were obtained in accordance with Example 19, the purified rice-derived RGC1 mentioned above and the purified soybean-derived SGC1 mentioned above were each added to a 50 mM sodium acetate buffer solution (pH 5.5) containing 100 µM glucosylceramide (cerebroside B), 0.1% Tween 20, and 0.05% sodium cholate, and incubated at each temperature for 15 minutes. Subsequently, a 4-fold amount of ethanol was added to the obtained reaction solution, mixed therewith, and then centrifuged at 15000 rpm for 20 minutes. The supernatant was subjected to high-performance liquid chromatography analysis to measure the amount of ceramide generated through enzymatic reaction. The high-performance liquid chromatography analysis was carried out in the same manner as in Example 5. The amount of ceramide and enzymatic activity were also calculated in the same manner as in Example 5. As a result, a temperature that attained the highest glucocerebrosidase activity was regarded as the optimum temperature. This test was conducted three times, and an average value was determined therefrom. The results are shown in Table 10 below.

**[Table 10]**

| | **Optimum temperature (°C)** |
|---|---|
| **Human-derived glucocerebrosidase (imiglucerase)** | **42.5** |
| **Rice-derived glucocerebrosidase (RGC1)** | **54.0** |
| **Maize-derived glucocerebrosidase** | **55.5** |
| **Wheat-derived glucocerebrosidase** | **51.6** |
| **Tulip-derived glucocerebrosidase** | **47.7** |
| **Narcissus-derived glucocerebrosidase** | **48.7** |
| **Morning star lily-derived glucocerebrosidase** | **54.4** |
| **Arabidopsis thaliana-derived glucocerebrosidase** | **53.9** |
| **Cabbage-derived glucocerebrosidase** | **56.3** |
| **Japanese radish-derived glucocerebrosidase** | **54.2** |
| **Rapeseed-derived glucocerebrosidase** | **53.1** |
| **Bitter cucumber-derived glucocerebrosidase** | **60.4** |
| **Melon-derived glucocerebrosidase** | **63.4** |
| **Cucumber-derived glucocerebrosidase** | **64.0** |
| **Lettuce-derived glucocerebrosidase** | **54.6** |
| **Gerbera-derived glucocerebrosidase** | **62.6** |
| **Gazania-derived glucocerebrosidase** | **57.5** |
| **Goldenmane tickseed-derived glucocerebrosidase** | **57.5** |
| **Tomato-derived glucocerebrosidase** | **54.2** |
| **Eggplant-derived glucocerebrosidase** | **53.7** |
| **Tobacco-derived glucocerebrosidase** | **56.8** |
| **Cherry-derived glucocerebrosidase** | **56.6** |
| **Rose-derived glucocerebrosidase** | **61.7** |
| **Lupine-derived glucocerebrosidase** | **50.6** |
| **Japanese wisteria-derived glucocerebrosidase** | **56.8** |
| **Broad bean-derived glucocerebrosidase** | **62.5** |
| **Soybean-derived glucocerebrosidase (SGC1)** | **64.0** |

From the results, glucocerebrosidase derived from many seed plants including rice and soybean was also found to have a higher optimum temperature than that of imiglucerase, human-derived glucocerebrosidase. As shown in Examples 6 and 13, for enzymes, it is generally known that a higher optimum temperature leads to better thermostability. Therefore, it can be concluded that seed plant-derived glucocerebrosidase belonging to GH1 is superior in stability against heat to human-derived glucocerebrosidase imiglucerase, that is, has thermostability.

This application claims the priority based on Japanese Patent Application No. 2020-88950 filed on May 21, 2020, the disclosure of which is incorporated herein

## Claims

1. A protein which is derived from a plant, belongs to glycoside hydrolase family 1, and has glucocerebrosidase activity.

2. The protein according to claim 1, wherein the plant is a seed plant.

3. The protein according to claim 2, wherein the seed plant is one of a plant of the family *Brassicaceae,* a plant of the family *Poaceae,* a plant of the family *Cucurbitaceae,* a plant of the family *Compositae,* a plant of the family *Solanaceae,* a plant of the family *Rosaceae,* a plant of the family *Amaryllidaceae*, a plant of the family *Leguminosae,* and a plant of the family *Liliaceae.*

4. A protein shown in the following (A), (B), or (C):
(A) a protein consisting of an amino acid sequence represented by SEQ ID NO: 1, amino acid positions 38 to 521 of SEQ ID NO: 1, SEQ ID NO: 2, or amino acid positions 19 to 503 of SEQ ID NO: 2 in the sequence listing.
(B) a protein which consists of the amino acid sequence represented by SEQ ID NO: 1, amino acid positions 38 to 521 of SEQ ID NO: 1, SEQ ID NO: 2, or amino acid positions 19 to 503 of SEQ ID NO: 2 in the sequence listing, having one or several nucleotides substituted, deleted, inserted, or added, belongs to glycoside hydrolase family 1, and has glucocerebrosidase activity and thermostability.
(C) a protein which has homology equal to or more than 60% to the amino acid sequence represented by SEQ ID NO: 1, amino acid positions 38 to 521 of SEQ ID NO: 1, SEQ ID NO: 2, or amino acid positions 19 to 503 of SEQ ID NO: 2 in the sequence listing, belongs to glycoside hydrolase family 1, and has glucocerebrosidase activity and thermostability.

5. DNA encoding a protein according to any one of claims 1 to 4.

6. DNA shown in the following (a), (b), or (c):
(a) DNA which consists of a nucleotide sequence represented by SEQ ID NO: 3, nucleotide positions 112 to 1566 of SEQ ID NO: 3, SEQ ID NO: 4, or nucleotide positions 55 to 1512 of SEQ ID NO: 4 in the sequence listing, and encodes a protein belonging to glycoside hydrolase family 1 and having glucocerebrosidase activity.
(b) DNA which consists of the nucleotide sequence represented by SEQ ID NO: 3, nucleotide positions 112 to 1566 of SEQ ID NO: 3, SEQ ID NO: 4, or nucleotide positions 55 to 1512 of SEQ ID NO: 4 in the sequence listing, having one or several nucleotides substituted, deleted, inserted, or added, and encodes a protein belonging to glycoside hydrolase family 1 and having glucocerebrosidase activity and thermostability.
(c) DNA which is capable of hybridizing under stringent conditions to DNA consisting of a nucleotide sequence complementary to the nucleotide sequence represented by SEQ ID NO: 3, nucleotide positions 112 to 1566 of SEQ ID NO: 3, SEQ ID NO: 4, or nucleotide positions 55 to 1512 of SEQ ID NO: 4 in the sequence listing, and encodes a protein belonging to glycoside hydrolase family 1 and having glucocerebrosidase activity and thermostability.

7. An expression vector for the expression of a protein belonging to glycoside hydrolase family 1 and having glucocerebrosidase activity and thermostability, the expression vector comprising DNA according to claim 5 or 6.

8. A transformant harboring an expression vector according to claim 7.

9. The transformant according to claim 8, wherein the transformant is one of a plant, a plant cell, an animal cell, *Escherichia coli,* a yeast, and a filamentous fungus.

10. A method for producing a protein, comprising the steps of: breeding or culturing a transformant according to claim 8 or 9; and recovering a protein according to any one of claims 1 to 4 from the transformant or a material containing the transformant obtained by the step.

11. An enzyme composition for glucosylceramide hydrolysis, comprising a protein according to any one of claims 1 to 4.

12. A pharmaceutical composition comprising a protein according to any one of claims 1 to 4 as an active ingredient.

13. The pharmaceutical composition according to claim 12 for Gaucher's disease prevention and treatment.

14. A food composition comprising a protein according to any one of claims 1 to 4, and glucosylceramide isolated from a plant, an animal, or a microbe, or chemically synthesized glucosylceramide.

15. A method for producing ceramide, comprising the step of using a protein according to any one of claims 1 to 4 to generate ceramide from glucosylceramide isolated from a plant, an animal, or a microbe, or chemically synthesized glucosylceramide.


[Sequence Listing]
<110> Teikyo University
<120> A thermostable glucocerebrosidase
<l30> FTK-0001WO
<160> 24

<210> 1
<211> 521
<212> PRT
<2l3> Oryza sativa L. cv. Nipponbare
<400> 1

<210> 2

<211> 503

<212> PRT

<213> Glycine max (L.) Merr. Enrei

<400> 2

Met Ala Phe Arg Gly Gly Thr Met Leu Ile Leu Thr Met Met Ala Leu

                5                   10                  15

Leu Glu Ile Gln Ile Cys Ser Ser Glu Ile Asn Arg Gly Asn Phe Pro

            20                  25                  30

Asn Gly Phe Val Phe Gly Thr Ala Ser Ser Ala Phe Gln Tyr Glu Gly

        35                  40                  45

Ala Val Lys Glu Asp Gly Arg Gly Pro Ser Val Trp Asp Thr Phe Ser

    50                  55                  60

His Thr Phe Gly Lys Ile Ile Asp Phe Ser Asn Ala Asp Val Ala Val

65                  70                  75                  80

Asp Gln Tyr His Arg Tyr Glu Glu Asp Ile Gln Leu Met Lys Asp Met

                85                  90                  95

Gly Met Asp Ala Tyr Arg Phe Ser Ile Ser Trp Ser Arg Ile Phe Pro

            100                 105                 110

Asn Gly Tyr Gly Gln Ile Asn Gln Ala Gly Val Asp His Tyr Asn Lys

        115                 120                 125

Leu Ile Asn Ala Leu Leu Ala Lys Gly Ile Glu Pro Tyr Val Thr Leu

    130                 135                 140

Tyr His Trp Asp Leu Pro Gln Ala Leu Glu Asn Lys Tyr Ser Gly Trp

145                 150                 155                 160

Leu Asn Ala Ser Ile Ile Met Asp Phe Ala Thr Tyr Ala Glu Thr Cys

                165                 170                 175

Phe Gln Lys Phe Gly Asp Arg Val Lys His Trp Ile Thr Phe Asn Glu

            180                 185                 190

Pro His Thr Phe Ala Thr Gln Gly Tyr Asp Val Gly Leu Gln Ala Pro

        195                 200                 205
Gly Arg Cys Ser Ile Leu Leu His Leu Phe Cys Arg Ala Gly Asn Ser
    210                 215                 220
Ala Thr Glu Pro Tyr Ile Val Ala His Asn Val Leu Leu Ser His Ala
225                 230                 235                 240
Thr Val Ala Asp Ile Tyr Arg Lys Lys Tyr Lys Lys Ile Gln Gly Gly
                245                 250                 255
Ser Leu Gly Val Ala Phe Asp Val Ile Trp Tyr Glu Pro Leu Thr Asn
            260                 265                 270
Thr Lys Glu Asp Ile Asp Ala Ala Gln Arg Ala Gln Asp Phe Gln Leu
        275                 280                 285
Gly Trp Phe Leu Asp Pro Leu Met Phe Gly Asp Tyr Pro Ser Ser Met
    290                 295                 300
Arg Thr Arg Val Gly Ser Arg Leu Pro Lys Phe Ser Gln Ser Glu Ala
305                 310                 315                 320
Ala Leu Val Lys Gly Ser Leu Asp Phe Val Gly Ile Asn His Tyr Thr
                325                 330                 335
Thr Phe Tyr Ala Lys Asp Asn Ser Thr Asn Leu Ile Gly Thr Leu Leu
            340                 345                 350
His Asp Ser Ile Ala Asp Ser Gly Ala Val Thr Leu Pro Phe Asn Gly
        355                 360                 365
Thr Lys Ala Ile Ser Glu Arg Ala Ser Ser Ile Trp Leu Tyr Ile Val
    370                 375                 380
Pro Gln Ser Met Arg Ser Leu Met Ile Tyr Ile Lys Gln Lys Tyr Gly
385                 390                 395                 400
Asn Pro Pro Val Tyr Ile Thr Glu Asn Gly Met Asp Asp Pro Asn Ser
                405                 410                 415

Ile Phe Ile Ser Ile Lys Asp Ala Leu Lys Asp Glu Lys Arg Ile Arg
            420                 425                 430
Tyr His Thr Gly Tyr Leu Ser Tyr Leu Leu Ala Ser Ile Lys Asp Gly
        435                 440                 445
Cys Asn Val Lys Gly Tyr Phe Val Trp Ser Leu Leu Asp Asn Trp Glu
    450                 455                 460
Trp Ser Ala Gly Tyr Thr Ser Arg Phe Gly Leu Tyr Phe Val Asp Tyr
465                 470                 475                 480
Lys Asp Asn Leu Lys Arg Tyr Pro Lys Gln Ser Val Glu Trp Phe Lys
                485                 490                 495
Asn Phe Leu Lys Pro Thr Lys
            500

<210> 3
<211> 1566
<212> DNA
<213> Oryza sativa L. cv. Nipponbare
<400> 3
atggggagga taaagagtag tagtgggcga tgcagcacgg cgaggctgga ggcggtcgcc 60
gtcctcgtcg tcgtcttcgg cgtcgcgtcg tcgtcgctgc gaggatgcat tgcgcagcag 120
agcggaggag ggctaaccag gggcagcttc cccgaggggt tcgtcttcgg caccgcctcc 180
gccgcgtacc agtacgaggg agctgtgaag gaggacggga gagggcagac catctgggac 240
acgttcgcgc acacctttgg aaagatcacc gacttcagca atgctgatgt tgcagttgat 300
cagtaccacc gtttcgagga ggatatacag ctcatggcag acatggggat ggatgcgtat 360
cggttctcga tagcatggtc aagaatctac ccaaatggtg ttggtcaagt caatcaagct 420
ggtatcgacc actacaacaa gctgatcgat gcacttctag caaaaggaat tcagccatat 480
gtgacactct accactggga cctcccccag gcccttgaag acaagtacaa gggctggctt 540

gacaggcaga tagtggacga tttcgcggcg tacgcggaga cgtgcttcag ggagttcggg 600

gacagggtga agcactggat cacgctcaac gagccgcaca cggtggccat ccagggctac 660

gacgcagggc tccaggcccc cggccgctgc tccgtgctgc tccacctcta ctgcaaggcc 720

ggcaactccg gcaccgagcc ctacgtcgtc gcccaccact tcatcctcgc ccacgccgcc 780

gccgccagca tctacaggac aaaatacaag gcgacgcaga acgggcagct tgggatagcg 840

ttcgacgtga tgtggttcga gccgatgtcc aacaccacga tcgacatcga ggcggccaag 900

agagcgcagg agtttcagct aggatggttt gctgatccgt tcttcttcgg cgactacccg 960

gcgacgatga gggcgagggt gggggagagg ctgccgaggt tcacggcgga tgaggccgcc 1020

gtcgtcaagg gggcgctgga tttcgtcggc ataaaccact acaccaccta ctacacgagg 1080

cacaacaaca ccaacatcat cgggacattg ctcaacaaca ccttggcaga caccggcacc 1140

gtcagcctcc cattcaagaa tgggaagcca attggagata gggcaaattc gatatggctg 1200

tacattgtgc cccgagggat gaggagcctg atgaactatg tcaaggaaag gtacaacagc 1260

ccaccagtgt acatcactga aaacgggatg gatgacagca acaacccgtt catttccatc 1320

aaggacgccc tcaaggacag caagaggatc aaataccaca atgactacct caccaatctg 1380

gctgcttcca tcaaggagga cgggtgcgac gtacgtgggt acttcgcgtg gtctctgctg 1440

gacaactggg agtgggcggc cggatactcc tcgagattcg ggctctactt cgtggactac 1500

aaggataacc tcaagagata ccccaagaac tcggtgcagt ggttcaaggc cctcctgaag 1560

acctga 1566

<210> 4

<211> 1512

<212> DNA

<213> Glycine max (L.) Merr. Enrei

<400> 4

atggcgttta gaggaggcac tatgttgata ttaacaatga tggcattact tgagattcag 60

atatgctcat cggagataaa ccgtggaaac tttccaaatg gcttcgtatt tggcactgcc 120

tcttcagctt ttcagtatga aggggcagtg aaagaagacg gaaggggacc ctctgtgtgg 180

gacacttttt cacatacttt tggcaaaata attgatttca gcaatgctga tgttgcggtg    240

gatcagtacc accgatacga agaagatata caactgatga aggacatggg aatggatgcc    300

tatagatttt ccatttcttg gtctcggatt tttcccaatg gatatggcca aatcaatcag    360

gcaggagttg atcattataa taaactcatc aacgcgttac tagccaaagg aattgaacca    420

tatgtgactc tctatcactg ggacctgcct caagccttgg aaaacaagta cagtggatgg    480

ctaaacgctt caatcataat ggactttgca acttatgctg agacatgctt tcagaaattt    540

ggggataggg tgaagcattg gatcacattc aatgagccac atacatttgc cacccaagga    600

tatgatgttg gtctacaagc gcctggtcga tgctccattc tccttcacct gttttgtagg    660

gcggggaact ctgctactga accttacatt gttgcacaca atgtcctact ctctcatgca    720

acagtagcag atatttatag gaaaaaatat aagaaaatac agggaggatc actaggggta    780

gcttttgatg tcatttggta tgagccacta acaaatacca aagaagacat tgatgcagct    840

caaagagctc aagattttca gctgggctgg tttcttgatc ctttgatgtt tggggattat    900

ccaagctcaa tgaggactag agtaggaagc aggctaccga aattttcgca atcagaagct    960

gctcttgtta agggttcatt agattttgtt ggaatcaatc attacaccac attttatgca   1020

aaagacaatt ctactaattt aattggaacc ctgctccatg attccattgc agactctggt   1080

gccgttaccc ttccattcaa tggtactaaa gctatttcgg aaagggcaag ttctatatgg   1140

ctgtatattg tgccacaaag catgagaagc ttaatgatct atatcaaaca aaagtatgga   1200

aaccctcctg tctatattac agaaaatggg atggatgatc caaatagtat atttatctcc   1260

attaaggatg ctctaaagga tgaaaaacgg atcagatacc acactggcta tttatcttac   1320

ttgttggctt ctatcaaaga tggttgcaat gtgaaaggtt attttgtgtg gtcactattg   1380

gataactggg agtggtcagc tggatacacg tctagatttg gtctatattt tgttgactac   1440

aaagacaacc tgaagagata tcccaaacaa tcagtggaat ggttcaagaa cttcttgaaa   1500

cctactaaat aa                                                       1512

<210> 5

<211> 130

<212> PRT

<213> Oryza sativa L. cv. Nipponbare

<400> 5

Gly Ser Phe Pro Glu Gly Phe Val Phe Gly Thr Ala Ser Ala Ala Tyr

                5                   10                  15

Gln Tyr Glu Gly Ala Val Lys Glu Asp Gly Arg Gly Gln Thr Ile Trp

            20                  25                  30

Asp Thr Phe Ala His Thr Phe Gly Lys Ile Thr Asp Phe Ser Asn Ala

        35                  40                  45

Asp Val Ala Val Asp Gln Tyr His Arg Phe Glu Glu Asp Ile Gln Leu

    50                  55                  60

Met Ala Asp Met Gly Met Asp Ala Tyr Arg Phe Ser Ile Ala Trp Ser

65                  70                  75                  80

Arg Ile Tyr Pro Asn Gly Val Gly Gln Val Asn Gln Ala Gly Ile Asp

                85                  90                  95

His Tyr Asn Lys Leu Ile Asp Ala Leu Leu Ala Lys Gly Ile Gln Pro

            100                 105                 110

Tyr Val Thr Leu Tyr His Trp Asp Leu Pro Gln Ala Leu Glu Asp Lys

        115                 120                 125

Tyr Lys

    130

<210> 6

<211> 15

<212> PRT

<213> Oryza sativa L. cv. Nipponbare

<400> 6

Gln Ile Val Asp Asp Phe Ala Ala Tyr Ala Glu Thr Cys Phe Arg

                5                   10                  15

<210> 7

<211> 23

<212> PRT

<2l3> Oryza sativa L. cv. Nipponbare

<400> 7

Ala Gln Glu Phe Gln Leu Gly Trp Phe Ala Asp Pro Phe Phe Phe Gly

                5                   10                  15

Asp Tyr Pro Ala Thr Met Arg

            20

<210> 8

<211> 17

<212> PRT

<2l3> Oryza sativa L. cv. Nipponbare

<400> 8

Gly Ala Leu Asp Phe Val Gly Ile Asn His Tyr Thr Thr Tyr Tyr Thr

                5                   10                  15

Arg

<210> 9

<211> 11

<212> PRT

<2l3> Oryza sativa L. cv. Nipponbare

<400> 9

Ala Asn Ser Ile Trp Leu Tyr Ile Val Pro Arg
                5                   10

<210> 10
<211> 7
<212> PRT
<2l3> Oryza sativa L. cv. Nipponbare
<400> 10
Ser Leu Met Asn Tyr Val Lys
                5

<210> 11
<211> 24
<212> PRT
<2l3> Oryza sativa L. cv. Nipponbare
<400> 11
Tyr Asn Ser Pro Pro Val Tyr Ile Thr Glu Asn Gly Met Asp Asp Ser
                5                   10                  15
Asn Asn Pro Phe Ile Ser Ile Lys
            20

<210> 12
<211> 54
<212> PRT
<2l3> Oryza sativa L. cv. Nipponbare
<400> 12
Tyr His Asn Asp Tyr Leu Thr Asn Leu Ala Ala Ser Ile Lys Glu Asp

                5                   10                  15
Gly Cys Asp Val Arg Gly Tyr Phe Ala Trp Ser Leu Leu Asp Asn Trp
            20                  25                  30
Glu Trp Ala Ala Gly Tyr Ser Ser Arg Phe Gly Leu Tyr Phe Val Asp
        35                  40                  45
Tyr Lys Asp Asn Leu Lys
    50

<210> 13
<211> 7
<212> PRT
<2l3> Oryza sativa L. cv. Nipponbare
<400> 13
Asn Ser Val Gln Trp Phe Lys
                5

<210> 14
<211> 51
<212> DNA
<213> Artificial sequence
<220>
<223> F-primer for amplifying Oryza sativa L. cv. Nipponbare Os3BGlu6 gene
sequence
<400> 14
tgattacgcc aagctgaagg agatatacat atggcgcagc agagcggagg a 51

<210> 15

<211> 32

<212> DNA

<213> Artificial sequence

<220>

<223> R-primer for amplifying Oryza sativa L. cv. Nipponbare Os3BGlu6 gene

sequence

<400> 15

caggcatgca agcttcaggt cttcaggagg gc                                    32

<210> 16

<211> 66

<212> PRT

<2l3> Glycine max (L.) Merr. Enrei

<400> 16

Gly Asn Phe Pro Asn Gly Phe Val Phe Gly Thr Ala Ser Ser Ala Phe

                5                   10                  15

Gln Tyr Glu Gly Ala Val Lys Glu Asp Gly Arg Gly Pro Ser Val Trp

            20                  25                  30

Asp Thr Phe Ser His Thr Phe Gly Lys Ile Ile Asp Phe Ser Asn Ala

        35                  40                  45

Asp Val Ala Val Asp Gln Tyr His Arg Tyr Glu Glu Asp Ile Gln Leu

    50                  55                  60

Met Lys

65

<210> 17

<211> 7

<212> PRT

<2l3> Glycine max (L.) Merr. Enrei

<400> 17

Phe Ser Ile Ser Trp Ser Arg

                5

<210> 18

<211> 20

<212> PRT

<2l3> Glycine max (L.) Merr. Enrei

<400> 18

Gly Ile Glu Pro Tyr Val Thr Leu Tyr His Trp Asp Leu Pro Gln Ala

                5                   10                  15

Leu Glu Asn Lys

            20

<210> 19

<211> 27

<212> PRT

<2l3> Glycine max (L.) Merr. Enrei

<400> 19

Ala Gly Asn Ser Ala Thr Glu Pro Tyr Ile Val Ala His Asn Val Leu

                5                   10                  15

Leu Ser His Ala Thr Val Ala Asp Ile Tyr Arg

            20                  25

<210> 20

<211> 53

<212> PRT

<2l3> Glycine max (L.) Merr. Enrei

<400> 20

Ile Gln Gly Gly Ser Leu Gly Val Ala Phe Asp Val Ile Trp Tyr Glu

                5                   10                  15

Pro Leu Thr Asn Thr Lys Glu Asp Ile Asp Ala Ala Gln Arg Ala Gln

            20                  25                  30

Asp Phe Gln Leu Gly Trp Phe Leu Asp Pro Leu Met Phe Gly Asp Tyr

        35                  40                  45

Pro Ser Ser Met Arg

    50

<210> 21

<211> 17

<212> PRT

<2l3> Glycine max (L.) Merr. Enrei

<400> 21

Gly Ser Leu Asp Phe Val Gly Ile Asn His Tyr Thr Thr Phe Tyr Ala

                5                   10                  15

Lys

<210> 22

<211> 13

<212> PRT

<2l3> Glycine max (L.) Merr. Enrei

<400> 22

Phe Gly Leu Tyr Phe Val Asp Tyr Lys Asp Asn Leu Lys

                5                   10

<210> 23

<211> 52

<212> DNA

<213> Artificial sequence

<220>

<223> F-primer for amplifying Glycine max (L.) Merr. Enrei beta-glucosidase 40

gene sequence

<400> 23

tgattacgcc aagctgaagg agatatacat atgattcaga tatgctcatc gg 52

<210> 24

<211> 36

<212> DNA

<213> Artificial sequence

<220>

<223> R-primer for amplifying Glycine max (L.) Merr. Enrei beta-glucosidase 4

0 gene sequence

<400> 24

caggcatgca agctttattt agtaggtttc aagaag 36